# EUROPEAN PATENT APPLICATION

(11) **EP 4 410 967 A1**
(43) Date of publication of application: **07.08.2024**
(21) Application number: 22876223.3
(22) Date of filing: 27.09.2022
(51) Int. Cl.: C12N 5/10, C12M 1/00

(54) **METHOD FOR PRODUCING PLURIPOTENT STEM CELLS**

(30) Priority: 28.09.2021 WO PCT/JP2021/035693
(71) Applicant: Cira Foundation, Kyoto-shi, Kyoto 606-8397 (JP)
(72) Inventor: TSUKAHARA, Masayoshi, Kyoto-shi, Kyoto 606-8397 (JP); YAMAMOTO, Yuko, Kyoto-shi, Kyoto 606-8397 (JP)
(74) Representative: Hoffmann Eitle
(86) International application number: PCT/JP2022/035937
(87) International publication number: WO 2023/054357

(57) **Abstract**

Provided is a method for producing pluripotent stem cells from somatic cells, comprising the steps of(1) seeding somatic cells in a container comprising at least one compartment configured to permit gathering two or more somatic cells; (2) bringing a reprogramming factor into contact with the somatic cells; and (3) culturing the somatic cells in contact with the reprogramming factor in a state where the two or more somatic cells are gathered in the compartment.

## Description

### TECHNICAL FIELD

The present application relates to a method for producing pluripotent stem cells from somatic cells, particularly, blood cells.

### BACKGROUND ART

iPS cells, which are pluripotent stem cells produced by reprogramming somatic cells, can be differentiated into various cells and are expected to be used in regenerative medicine, such as medical transplantation. Methods for producing pluripotent stem cells using somatic cells from various origins are known.

Producing pluripotent stem cells from blood cells, particularly, peripheral blood mononuclear cells, is advantageous in that it is less invasive and less stressful for the patient. However, the reprogramming efficiency is quite low, and methods to improve the efficiency have been demanded. In addition, blood cells do not adhere to the bottom surface of a plastic petri dish or the like and can be advantageously grown in suspension culture. Blood cells also have an advantage in ease in handling in culture using an automatic culture apparatus or the like.

### CITATION LIST

### PATENT LITERATURE

Patent Literature 1: JP2018-23401A
Patent Literature 2: JP2016-135102A
Patent Literature 3: JP2021-035399A
Patent Literature 4: JP2020-036608A
Patent Literature 5: JP2018/143243A
Patent Literature 6: JP2017/110724A

### SUMMARY OF INVENTION

### TECHNICAL PROBLEM

An object of the present application is to provide a method for producing pluripotent stem cells.

### SOLUTION TO PROBLEM

The present application provides the following embodiments.
[1] A method for producing pluripotent stem cells from somatic cells, comprising the steps of:
   (1) seeding somatic cells in a container comprising at least one compartment configured to permit gathering two or more somatic cells;
   (2) bringing a reprogramming factor into contact with the somatic cells; and
   (3) culturing the somatic cells in contact with the reprogramming factor in a state where the two or more somatic cells are gathered in the at least one compartment.
[2] The method according to [1], wherein the compartment has a gathering portion with low cell adhesion configured to permit gathering two or more somatic cells; and
   the method further comprises the step of gathering two or more somatic cells in the gathering portion.
[3] The method according to [2], wherein the gathering step includes at least one action selected from rotation, rocking and shaking of the container.
[4] The method according to [3], wherein the container has a plurality of surfaces, and
   the container is rotated about a rotation axis that is parallel to or intersecting a first surface having the largest area of the plurality of surfaces.
[5] The method according to [4], wherein an angle of the rotation axis with respect to the first surface is a right angle.
[6] The method according to [5], wherein the two or more somatic cells are aggregated in a region closer to the rotation axis than the center of the container in a radial direction with respect to the rotation axis.
[7] The method according to [5], wherein the two or more somatic cells are gathered in a region more distant from the rotation axis than the center of the container in a radial direction with respect to the rotation axis.
[8] The method according to any one of [1] to [7], wherein the at least one compartment comprises:
   a recessed portion having an opening provided on a surface of the container and a bottom surface with low cell adhesion, and
   wherein the recessed portion comprises
   a gathering portion configured to permit gathering two or more somatic cells; and
   a guide portion configured to guide the somatic cells flowing into the container to the gathering portion.
[9] The method according to [8], wherein the guide portion is an inclined surface which is inclined with respect to a surface of the container.
[10] The method according to [9], wherein an angle formed between the inclined surface and the surface of the container is 15° to 60°.
[11] The method according to [9] or [10], wherein the at least one inclined surface is configured with a flat surface.
[12] The method according to [9] or [10], wherein the at least one inclined surface is configured with a curved surface.
[13] The method according to any one of [9] to [12], wherein the guide portion has two or more inclined surfaces.
[14] The method according to any one of [9] to [11], wherein the inclined surface is formed with a polygonal surface.
[15] The method according to [14], wherein the recessed portion is shaped like a polygonal cone that is configured with three or more inclined surfaces arranged adjacent to each other and tapering from the opening to the bottom, and
   the bottom surface constitutes the gathering portion, and the side surface of the recessed portion constitutes the guide portion.
[16] The method according to [14], wherein the inclined surface is substantially triangular.
[17] The method according to [12], wherein the recessed portion is substantially shaped like a cone tapering from the opening to the bottom, and
   the bottom surface constitutes the gathering portion, and the side surface of the recessed portion constitutes the guide portion.
[18] The method according to any one of [1] to [17], wherein the container comprises the two or more compartments.
[19] The method according to any one of [1] to [18], wherein the somatic cells are floating somatic cells.
[20] The method according to [19], wherein the floating somatic cells are blood cells.
[21] The method according to [20], wherein the blood cells are peripheral blood mononuclear cells.
[22] The method according to [20], wherein the blood cells comprise hematopoietic progenitor cells.
[23] The method according to [20], wherein the blood cells are CD34-positive cells.
[24] The method according to any of [20] to [23], wherein the blood cells are derived from peripheral blood or cord blood.
[25] The method according to any of [1] to [24], wherein the somatic cells are gathered at one point in the compartment in step (3).
[26] The method according to [25], wherein the somatic cells are seeded at 50 to 400 cells/compartment.
[27] The method according to [26], wherein the somatic cells are seeded at 100 to 300 cells/compartment.
[28] The method according to any of [1] to [13] and [18] to [24], wherein the somatic cells are gathered linearly in step (3).
[29] The method according to [28], wherein the somatic cells are seeded so that a theoretical value obtained by calculating the number of somatic cells accumulated on a cross section perpendicular to a linear portion is about 100 to 1,000.
[30] The method according to any one of [1] to [29], further including the step of expanding somatic cells prior to bringing the somatic cells into contact with the reprogramming factor.
[31] The method according to any one of [1] to [30], wherein in step (2) the reprogramming factor is brought into contact with the somatic cells by using a Sendai virus vector.
[32] The method according to any one of [1] to [31], wherein the reprogramming factor is an RNA.
[33] The method according to any one of [1] to [32], wherein the somatic cells are human somatic cells.
[34] The method according to any one of [1] to [33], wherein step (2) is carried out after step (1).
[35] The method according to any one of [1] to [33], wherein step (1) is carried out after step (2).
[36] A container used for the method according to any one of [1] to [35], wherein the container has the compartment capable of gathering two or more somatic cells.
[37] A container comprising
   a container body capable of containing somatic cells; and
   at least one compartment disposed within the container body,
   wherein the compartment comprises
      a gathering portion with low cell adhesion capable of gathering two or more somatic cells; and
      a guide portion that guides somatic cells having flown into the container to the gathering portion.
[38] The container according to [37], further comprises a passage portion which allows the inside of the container body and the outside of the container body to communicate with each other and allows somatic cells to pass therethrough.
[39] The container according to [37] or [38],
   wherein the compartment comprises an opening provided on the inner surface of the container body and a recessed portion having a bottom surface with low cell adhesion, and
   wherein the guide portion is an inclined surface constituting a part of the recessed portion and inclined with respect to the inner surface of the container body.
[40] The container according to [38], wherein the compartment comprises an opening provided on the inner surface of the container body and a recessed portion having a bottom surface with low cell adhesion, and
   the guide portion is a protruding portion which is adjacent to the opening outside the recessed portion, is located more distant from the passage portion than the opening, and protrudes from the inner surface.
[41] The container according to any one of [36] to [40], wherein the reprogramming factor is contained within the compartment.
[42] A system comprising
   the container according to any one of [36] to [40];
   a seeding device that supplies somatic cells to the container and seeds the somatic cells;
   a collecting device that discharges somatic cells in contact with a reprogramming factor from the inside of the container to the outside of the container and collects the somatic cells; and
   a controller that controls the seeding device and the collecting device.
[43] The system according to [42], further comprising a tilting device that tilts a portion of the container or the entire container with respect to a reference surface to cause the two or more somatic cells in the container to gather in the compartment.
[44] The system according to [43],
   wherein the container has a flexible bottom, and
   wherein the tilting device deforms the bottom to tilt the bottom surface with respect to the reference surface.
[45] The system according to [44], wherein the tilting device tilts the container by rotating the container about a rotation axis extending in a direction orthogonal to the reference surface, or tilts a part of the container or the entire container with respect to the reference surface by pressing a part of the bottom of the container.
[46] The system according to any one of [42] to [45], further comprising a shaking device that rocks or shakes the container enabling to cause the two or more somatic cells in the container to gather in the compartment.
[47] The system according to any one of [42] to [46], comprising a rotating device that rotates the container about the rotation axis enabling to cause the two or more somatic cells in the container to gather in the compartment by a centrifugal force.
[48] A kit for producing pluripotent stem cells from somatic cells, the kit comprising
   a container with low cell adhesion, having one or more compartments each capable of gathering two or more somatic cells; and
   a reprogramming factor.
[49] A program causing a computer to perform the method according to any one of [1] to [35].
[50] A method for producing differentiated cells, comprising the steps of
   providing pluripotent stem cells produced by the method according to any one of [1] to [35]; and
   culturing the provided cells in a culture medium for inducing cell differentiation.
[51] The method according to [50], wherein the differentiated cells are cardiomyocytes.

### ADVANTAGEOUS EFFECTS OF INVENTION

The present application provides a method for producing pluripotent stem cells with high efficiency. Further, the present application provides a container, a system, a kit, and a program for producing pluripotent stem cells.

### BRIEF DESCRIPTION OF DRAWINGS

[Figure 1] Figure 1 shows the morphology of cells immediately after seeding on an Aggrewell(TM) plate.
[Figure 2] Figure 2 shows the morphology of control cells (left) and the cells seeded on the Aggrewell(TM) plate (right) on Day 4.
[Figure 3] Figure 3 shows the morphologies of control cells (left) and the cells seeded on the Aggrewell(TM) plate (right) on Day 10.
[Figure 4] Figure 4 shows a fluorescence microscopy image of cells seeded on the Aggrewell(TM) plate on Day 10. The upper left image shows a fluorescence microscopy image of GFP. The upper right image shows an immunostaining image using a TRA-1-60 antibody. The lower left image shows an image obtained by merging the fluorescence microscopy image of GFP, the immunostaining image using the TRA-1-60 antibody, and a bright field image. The lower right image shows an enlarged image of the lower left image.
[Figure 5] Figure 5 shows the morphology of a cell and a fluorescence microscopy image thereof on Day 16, the cell being seeded on the Aggrewell(TM) plate and subcultured on Day 11. The upper left image shows a fluorescence microscopy image of GFP. The upper right image shows an immunostaining image using a TRA-1-60 antibody. The lower left image shows an image obtained by merging the fluorescence microscopy image of GFP and the immunostaining image using the TRA-1-60 antibody. The lower right image shows a bright-field image.
[Figure 6] Figure 6 shows bright-field images of cell culture (left) on a 24-well plate (control) on Day 9 and cell culture (right) on the Aggrewell(TM) plate.
[Figure 7] Figure 7 shows the relationship between the number of PBMC cells seeded for each microwell and the number of living cells on Day 14.
[Figure 8] Figure 8 shows the relationship between the number of PBMC seeded for each microwell and the number of TRA-1-positive cells on Day 14.
[Figure 9] Figure 9 shows the relationship between the number of PBMC seeded for each microwell and a ratio of TRA-1-positive cells on Day 14.
[Figure 10] Figure 10 is a schematic diagram for explaining the gathering step included in the first embodiment of the present disclosure.
[Figure 11] Figure 11 is a schematic diagram for explaining the gathering step included in the second embodiment of the present disclosure.
[Figure 12] Figure 12 is a schematic diagram illustrating a container according to the first embodiment of the present disclosure.
[Figure 13] Figure 13 is a schematic diagram illustrating a container according to the second embodiment of the present disclosure.
[Figure 14] Figure 14 is a schematic diagram illustrating a container according to the third embodiment of the present disclosure.
[Figure 15] Figure 15 is a schematic diagram illustrating a container according to the fourth embodiment of the present disclosure.
[Figure 16] Figure 16 is a schematic diagram illustrating a container according to the fifth embodiment of the present disclosure.
[Figure 17] Figure 17 is a schematic diagram illustrating a container according to the sixth embodiment of the present disclosure.
[Figure 18] Figure 18 is a schematic diagram illustrating a container according to the seventh embodiment of the present disclosure.
[Figure 19] Figure 19 is a schematic diagram illustrating a container according to the eighth embodiment of the present disclosure.
[Figure 20] Figure 20 is a schematic diagram illustrating a container according to the ninth embodiment of the present disclosure.
[Figure 21] Figure 21 is a schematic diagram illustrating a container according to the tenth embodiment of the present disclosure.
[Figure 22] Figure 22 is a schematic diagram illustrating a container according to the eleventh embodiment of the present disclosure.
[Figure 23] Figure 23 is a schematic diagram illustrating a container according to the twelfth embodiment of the present disclosure.
[Figure 24] Figure 24 is a block diagram illustrating a system according to an embodiment of the present disclosure.
[Figure 25] Figure 25 shows flow cytometry analysis of the ratio of troponin-positive cells in the cardiomyocytes differentiated from the iPS cells produced by the method of the present application.

### DESCRIPTION OF THE EMBODIMENTS

In the present specification and the claims, a numeric value with "about" is intended to include a ±10% range of the value. For example, "about 20" includes "18 to 22." A numerical range includes all numeric values between both end points and the numeric values of both end points. "About" for a range is applied to both end points of the range. Thus, for example, "about 20 to 30" includes "18 to 33."

### [Method For Producing Pluripotent Stem Cells]

The present application provides a method for producing pluripotent stem cells from somatic cells, the method including the steps of
(1) seeding somatic cells in a container having at least one compartment configured to permit gathering two or more somatic cells;
(2) bringing a reprogramming factor into contact with the somatic cells; and
(3) culturing the somatic cells in contact with the reprogramming factor in a state where the two or more somatic cells are gathered in the compartment.

### (Somatic Cells)

In the present disclosure, somatic cells are not particularly limited and thus any somatic cells can be used. Examples of somatic cells include epithelial cells to be cornified such as cornified epidermal cells;, mucosal epithelial cells such as epithelial cells of a tongue epithelium;, exocrine gland epithelial cells such as mammary gland cells; hormone-secreting cells, such as adrenal medullary cells; cells for metabolism and storage such as hepatocytes; luminal epithelial cells constituting an interface boundary such as type I alveolar cells; the luminal epithelial cells of an inner duct such as vascular endothelial cells; cells having cilia with transport capability such as airway epithelial cells; extracellular matrix-secreting cells such as fibroblasts; contractile cells such as smooth muscle cells; cells for blood and an immune system such as peripheral blood mononuclear cells, umbilical cord blood, T lymphocytes; sensory cells such as rod cells; autonomic nervous system neurons such as cholinergic neurons; supporting cells for sensory organs and peripheral neurons, such as companion cells; nerve cells and glial cells for a central nervous system, such as astrocytes; pigment cells such as retinal pigment epithelial cells; and the precursor cells thereof including tissue precursor cells. The degree of cell differentiation and the age of the animal from which the cells are collected are not particularly limited. Even if undifferentiated precursor cells including somatic stem cells or finally differentiated mature cells are used, the cells can similarly be used as the origin of somatic cells according to the present application. Examples of undifferentiated precursor cells include neural stem cells, hematopoietic progenitor cells, mesenchymal stem cells, and tissue stem cells or somatic stem cells such as dental pulp stem cells. In an embodiment, the somatic cells may be floating cells such as blood cells, for example, peripheral blood mononuclear cells, hematopoietic progenitor cells, and CD34-positive cells. The blood cells originate from, for example, peripheral blood or cord blood.

In the present application, animals as a source of somatic cells are not limited. For example, mammals such as mouse, rat, hamster, guinea pig, cow, horse, pig, sheep, monkey, orangutan, chimpanzee, dog, cat, bird, and human are used. Primates are preferred, and human is more preferred.

### (Pluripotent Stem Cells)

In the present application, "pluripotent stem cells" are cells having both capability of differentiating into various cells and proliferating. In a known method, somatic cells are reprogrammed to produce pluripotent stem cells by causing a factor called a reprogramming factor to act on the somatic cells.

### (Culture Medium)

In the present application, a culture medium may be prepared by properly adding required factors to a basal medium used for culturing animal cells. Examples of basal medium include IMDM, Medium 199, Eagle's Minimum Essential Medium (EMEM), αMEM, MEM Zinc Option, IMEM Zinc Option, Dulbecco's modified Eagle's Medium (DMEM), DMEM/F12, Ham's F12, RPMI 1640, Fischer's medium, and a mixture thereof. The basal medium may contain blood serum such as fetal bovine serum (FBS), or may be serum-free. The basal medium may contain, for example, one or more serum substitutes, such as albumin, insulin, transferrin, selenium, KnockOut Serum Replacement (KSR) (serum replacement during ES cell culture) (Invitrogen), N2 supplement(Invitrogen), B27 supplement (Invitrogen), a fatty acid, a collagen precursor, a trace element, 2-mercaptoethanol, and 3'-thiol glycerol or one or more substances including a lipid, an amino acid, L-glutamine, GlutaMAX (Invitrogen), a non-essential amino acid, a vitamin, a growth factor, a small molecule compound, an antibiotic such as streptomycin, penicillin, puromycin, mitomycin; an antioxidant, pyruvic acid, a buffering agent, a mineral, a cytokine, and an equivalent thereof. A commercially available culture medium may be used as the basal medium. For example, a medium such as StemSpan ACF (STEMCELL Technologies) or StemFit (R) AK03N medium (AJINOMOTO CO., INC.) is available.

### - Step (1)

In step (1), somatic cells are seeded in a container having at least one compartment configured to permit gathering two or more somatic cells.

### (Gathering of Somatic Cells)

"Gathering" or "gather" of somatic cells means a state where a somatic cell is in contact with at least one other somatic cell and a plurality of somatic cells are gathered in one place. A compartment that can gather two or more somatic cells is not particularly limited as long as two or more somatic cells can be gathered. For example, a compartment in which two or more somatic cells can be gathered at one place by a physical force, including a force generated by their own weight, may be used. Specifically, the two or more somatic cells in a compartment can be gathered by rotating, rocking, or shaking the container. A compartment in which an inclined surface extends from the opening to the bottom, which allows the cells to be gathered at the bottom by their own weight can also be employed. When a container having a compartment with an inclined surface extending from the opening to the bottom is used, the container may also be rotated, rocked or shaken so that the cells are gathered. Somatic cells may be gathered at a point (one or more points) or may be gathered linearly in the compartment.

In the present aspect, the container has one or more compartments, each is configured to permit gathering two or more somatic cells. The number of the compartments included in the container is not particularly limited and a person skilled in the art may appropriately determine the number. The container may have one, two, three, four, five, six, seven, eight, nine or ten compartments or one to one hundred thousand or more compartments. For example, if the container is a 24-well plate having 1200 compartments in each well, the number of compartments is 28,800. In one embodiment, the container has the two or more compartments.

In the present aspect, the compartment may have a low cell adhesion property. Examples of compartments with low cell adhesion property may include, but not limited to, a compartment in which no artificial treatment to enhance cell adhesion, such as coating with an extracellular matrix or the like is performed in a commercial cell culture container, and a compartment in which a treatment to suppress cell adhesion such as coating with polyhydroxyethyl methacrylate (poly-HEMA) or a polymer of 2-methacryloyloxyethyl is provided phosphorylcholine(Lipidure) but are not limited thereto.

The number of somatic cells seeded in step (1) may be appropriately determined by a person skilled in the art and is not particularly limited. For example, the number of cells may be determined such that most of the somatic cells can come into contact with at least one other somatic cell in step (3). When the somatic cells are gathered at a point in a compartment in step (3), the number of the cells may be 2 to 500 cells/compartment, 2 to 400 cells/compartment, or 3 to 300 cells/compartment. For example, the number of the cells may be 5 to 500 cells/compartment, 10 to 500 cells/compartment, 50 to 400 cells/compartment, or 100 to 300 cells/compartment, e.g., about 200 cells/compartment. When the somatic cells are linearly gathered in step (3), the number of the somatic cells to be seeded may be determined according to the length of the portion where somatic cells are gathered or the portion where somatic cells are likely to be gathered (also referred to as "linear portion"). The shape of the linear portion is not particularly limited and may be determined depending on the shape of the container. For example, the linear portion may be a straight line or a curved line. In one embodiment, somatic cells are seeded such that a theoretical value obtained by calculating the number of somatic cells accumulated on a cross section perpendicular to the linear portion is about one or more, about ten or more, about 100 or more, about 1,000 or more, or about 10,000 or more, for example, about one to 10,000 or about 100 to 1,000. The theoretical value is calculated by the following formula:
(the mean value of the diameters of the somatic cells seeded in a compartment) x (the number of the somatic cells seeded in the compartment)/(the length of the linear portion). For example, if the mean value of the diameters of the somatic cells is 10 µm and the length of the linear portion in the compartment is 10 cm, the number of the cells may be about 10⁴ cells or more/compartment, about 10⁵ cells or more/compartment, about 10⁶ cells or more/compartment, about 10⁷ cells or more/compartment, or about 10⁸ cells or more/compartment. For example, the number of cells may be about 10⁴ to 10⁸ cells/compartment or 10⁶ to 10⁷ cells/compartment.

### - Step (2)

In step (2), a reprogramming factor is brought into contact with the somatic cells.

### (Reprogramming factor)

In the present application, the reprogramming factor means a substance used alone or in cooperation with a plurality of factors to induce a differentiated state of certain cells to a less differentiated state. The reprogramming factors may include a factor necessary for nuclear reprogramming and an auxiliary factor (cofactor) for increasing the efficiency of nuclear reprogramming. The reprogramming factor may be, for example, a gene (DNA, RNA), a gene product (e.g., mRNA, miRNA, or protein), a small molecule compound, and a combination thereof.

When the reprogramming factor is a gene or a gene product thereof, the reprogramming factor is at least one selected from the group consisting of an Oct gene family gene, a Sox gene family gene, a Klf gene family gene, a Myc gene family gene, a Lin gene family gene, a Nanog gene, and a gene product thereof (WO 2007/69666, Japanese Patent No. 5696282, Science, 2007, 318:1917-1920). Among the genes, at least one selected from the group consisting of a gene of the Oct gene family, a gene of the Sox gene family, a gene of the Klf gene family, a gene of the Myc gene family, and a gene product thereof is particularly preferred.

Specific examples of the genes of these families and combinations thereof are listed below. Hereinafter, only the names of the genes are listed, and the use of their gene products is also included.

(a) A reprogramming factor composed of a gene of the Oct gene family;
(b) A combination of two reprogramming factors composed of a gene of the Oct gene family and a gene of the Sox gene family;
(c) A combination of two reprogramming factors composed of a gene of the Oct gene family and a gene of the Klf gene family;
(d) A combination of two reprogramming factors composed of a gene of the Oct gene family and a Nanog gene;
(e) A combination of three reprogramming factors composed of a gene of the Oct gene family, a gene of the Sox gene family, and a gene of the Klf gene family;
(f) A combination of three reprogramming factors composed of a gene of the Oct gene family, a gene of the Klf gene family, and a gene of the Myc gene family;
(g) A combination of four reprogramming factors composed of a gene of the Oct gene family, a gene of the Sox gene family, a gene of the Klf gene family, and a gene of the Myc gene family; and
(h) A combination of four reprogramming factors composed of a gene of the Oct gene family, a gene of the Sox gene family, a gene of the Lin gene family, and a Nanog gene.

More specifically, the following combinations are listed but are not limited thereto. In the following combinations, Sox2 gene may be replaced by Sox1 gene, Sox3 gene, Sox15 gene, Sox17 gene, or Sox18 gene. Klf4 gene may be replaced by Klfl gene, Klf2 gene, or Klf5 gene. c-Myc gene may be replaced by T58A (active variant) gene, N-Myc gene, or L-Myc gene.
(1) Oct3/4 gene, Klf4 gene, c-Myc gene
(2) Oct3/4 gene, Sox2 gene, Klf4 gene, c-Myc gene
(3) Oct3/4 gene, Sox2 gene, Klf4 gene, c-Myc gene, Fbx15 gene, Nanog gene, Eras gene, ECAT15-2 gene, Tell gene, β-catenin (active variant S33Y)
(4) Oct3/4 gene, Sox2 gene, Klf4 gene, c-Myc gene, hTERT gene, SV40Large T antigen (hereinafter, SV40LT) gene
(5) Oct3/4 gene, Sox2 gene, Klf4 gene, c-Myc gene, hTERT gene, HPV16E6 gene
(6) Oct3/4 gene, Sox2 gene, Klf4 gene, c-Myc gene, hTERT gene, HPV16E7 gene
(7) Oct3/4 gene, Sox2 gene, Klf4 gene, c-Myc gene, hTERT gene, HPV6E6 gene, HPV16E7 gene
(8) Oct3/4 gene, Sox2 gene, Klf4 gene, c-Myc gene, hTERT gene, Bmil gene See WO 2007/069666 for the combinations of (1) to (8), wherein for the combination of (2), see Nature Biotechnology, 26,101-106 (2008) for the replacement of Sox2 gene with Sox 18 gene and the replacement of Klf4 gene with Klf1 gene or Klf5 gene. See also, for example, Cell, 126, 663-676 (2006), Cell, 131, 861-872 (2007) for the combination of "Oct3/4 gene, Sox2 gene, Klf4 gene, c-Myc gene." See also Nat. Cell Biol., 11, 197-203 (2009) for the combination of "Oct3/4 gene, Sox2 gene, Klf2 (or Klf5) gene, c-Myc gene." See also Nature, 451, 141-146 (2008) for the combination of "Oct3/4 gene, Sox2 gene, Klf4 gene, c-Myc gene, hTERT gene, SV40LT gene."
(9) Oct3/4 gene, Sox2 gene, Klf4 gene (see Nature Biotechnology, 26, 101-106 (2008))
(10) Oct3/4 gene, Sox2 gene, Nanog gene, Lin28 gene (see Science, 318, 1917-1920 (2007))
(11) Oct3/4 gene, Sox2 gene, Nanog gene, Lin28 gene, hTERT gene, SV40LT gene (see Stem Cells, 26, 1998-2005 (2008))
(12) Oct3/4 gene, Sox2 gene, Klf4 gene, c-Myc gene, Nanog gene, Lin28 gene (see Cell Research (2008) 600-603)
(13) Oct3/4 gene, Sox2 gene, Klf4 gene, c-Myc gene, SV40LT gene (see Stem Cells, 26, 1998-2005 (2008))
(14) Oct3/4 gene, Klf4 gene (see Nature 454:646-650 (2008), Cell Stem Cell, 2:525-528(2008))
(15) Oct3/4 gene, c-Myc gene (see Nature 454:646-650 (2008))
(16) Oct3/4 gene, Sox2 gene (see Nature, 451, 141-146 (2008), WO 2008/118820)
(17) Oct3/4 gene, Sox2 gene, Nanog gene (see WO 2008/118820)
(18) Oct3/4 gene, Sox2 gene, Lin28 gene (see WO 2008/118820)
(19) Oct3/4 gene, Sox2 gene, c-Myc gene, Esrrb gene (an Essrrb gene may be replaced by an Esrrg gene, see Nat. Cell Biol., 11, 197-203 (2009))
(20) Oct3/4 gene, Sox2 gene, Esrrb gene (see Nat. Cell Biol., 11, 197-203 (2009))
(21) Oct3/4 gene, Klf4 gene, L-Myc gene
(22) Oct3/4 gene, Nagog gene
(23) Oct3/4 gene
(24) Oct3/4 gene, Klf4 gene, c-Myc gene, Sox2 gene, Nanog gene, Lin28 gene, SV40LT gene (see Science, 324: 797-801 (2009))

In the combinations of (1) to (24), the Oct3/4 gene can be replaced with another gene of Oct gene family (e.g., Oct1A or Oct6). The Sox2 gene (or Sox1 gene, Sox3 gene, Sox15 gene, Sox17 gene, or Sox18 gene) can be replaced with another gene of Sox gene family (e.g., Sox7 gene). The Lin28 gene can be replaced with another gene of Lin gene family (e.g., an Lin28b gene).

In addition, combinations including all the constituent elements of any one of the combinations (1) to (24) and further including any other substances may also be included in the category of "reprogramming factor" in the present application, although the combinations do not correspond to the combinations (1) to (24). Under the condition that somatic cells to be reprogrammed internally express some of the constituent elements in any one of the combinations (1) to (24) at a level sufficiently high for reprogramming, a combination of only other constituent elements than the constituent elements may also be included in the category of "reprogramming factor" in the present application.

In addition to the reprogramming factors discussed above, one or more reprogramming factors selected from the group consisting of Fbx15 gene, ERas gene, ECAT15-2 gene, Tcl1 gene, and β-catenin gene may be combined and/or one or more reprogramming factors selected from the group consisting of ECAT1 gene, Esg1 gene, Dnmt3L gene, ECAT8 gene, Gdf3 gene, Mybl2 gene, ECAT15-1 gene, Fthl17 gene, Sall4 gene, Rex1 gene, UTF1 gene, Stella gene, Stat3 gene, and Grb2 gene may also be combined. These combinations are specifically described in WO 2007/69666.

A preferable reprogramming factor is, for example, at least one selected from the group consisting of Oct3/4 gene, Sox2 gene, Klf4 gene, c-Myc gene (or L-Myc gene), Lin28 gene, Nanog gene, and a gene product thereof. Two or more genes are preferably selected and combined from the group consisting of Oct3/4 gene, Sox2 gene, Klf4 gene, c-Myc gene (or L-Myc gene), Lin28 gene, Nanog gene, and a gene product thereof, and three or more genes are more preferably selected and combined from the group. In particular, combinations of reprogramming factors to be preferably introduced may include at least (1) Oct3/4 gene or a gene product thereof, Sox2 gene or a gene product thereof, and Klf4 gene or a gene product thereof, (2) Oct3/4 gene or a gene product thereof, Sox2 gene or a gene product thereof, Klf4 gene or a gene product thereof, and c-Myc gene or a gene product thereof, (3) Oct3/4 gene or a gene product thereof, Sox2 gene or a gene product thereof, Klf4 gene or a gene product thereof, and L-Myc gene or a gene product thereof. Most of all, a combination of Oct3/4 gene or a gene product thereof, Sox2 gene or a gene product thereof, and Klf4 gene or a gene product thereof, and a combination of Oct3/4 gene or a gene product thereof, Sox2 gene or a gene product thereof, Klf4 gene or a gene product thereof, and L-Myc gene or a gene product thereof are preferable. Most preferably, a combination of Oct3/4 gene, Sox2 gene, and Klf4 gene, and a combination of Oct3/4 gene, Sox2 gene, Klf4 gene, and L-Myc gene are employed.

If the reprogramming factor is a gene or a gene product thereof, the origin of the gene is not particularly limited and any species may be selected according to the origin of the cells to be reprogrammed. Although human or another mammal such as mouse, rat, rabbit, pig, or a primate such as monkey may be selected, human is preferably selected.

cDNA sequence information of the reprogramming factors can be obtained from a known database. For example, an accession number in GenBank described in WO 2007/069666 may be referred to. Nanog gene is referred to as "ECAT4" in the publication.

Among the reprogramming factors, mouse and human cDNA sequence information of particularly preferable four genes (Oct3/4 gene, Sox2 gene, Klf4 gene, L-Myc gene) will be described below.
Gene name: Mouse/Human
Oct3/4: NM_013633/NM_002701
Sox2: NM_011443/NM_003106
Klf4: NM_010637/NM_004235
L-Myc: NM_008506/NM_001033081

cDNA of the reprogramming factors can be easily isolated from living cells as an origin of the sequence, by using a known method such as a PCR on the basis of the above cDNA sequence information or sequence information registered in a known database.

The sequences of the genes and the gene products of the reprogramming factors are not limited to the wild type. Any variations may be included if reprogramming of somatic cells can be induced. For example, a gene or mRNA encoding an amino acid sequence, in which one or a few e.g., three or less, five or less, ten or less, 15 or less, 20 or less, 25 or less of amino acids are added, deleted, substituted, and/or inserted to the above discussed reprogramming factor and capable of inducing reprogramming can be used in the present application. This also holds true for protein encoded by the gene or mRNA. If the biological activity (the ability to induce reprogramming) is maintained, variants of the above discussed polypeptides, such as polypeptides in which one to several residues (e.g., 2, 3, 4, 5, 6, 10, 15 or 20 residues) of amino acid at the N-terminus and/or the C-terminus are deleted or added, polypeptide in which one to several residues (e.g., 2, 3, 4, 5, 6, 10, 15 or 20 residues) of amino acid are substituted, and a gene or mRNA encoding the polypeptide may also be used. The variants may include a fragment, an analog, and a derivative of a natural protein and a protein fused with other polypeptides (e.g., foreign signal peptide or polypeptide with an added antigen fragment). In particular, the variants may include a polypeptide having a sequence obtained by substitution, deletion and/or addition of one or more amino acids of a wild-type amino acid sequence and having the same biological activity (e.g. activity for inducing reprogramming) as the wild-type protein. When a fragment of a wild-type protein is used, the fragment typically comprises a contiguous region which is 70% or more, preferably 80% or more or 85% or more, more preferably 90% or more, 95% or more or 98% of the wild-type polypeptide (mature type in the case of secretory protein).

The number of amino acid alterations is not particularly limited and is, for example, 30% or less of all amino acids of the natural mature polypeptide, preferably 25% or less, more preferably 20% or less, more preferably 15% or less, more preferably 10% or less, 5% or less, or 3% or less, and is, for example, 15 amino acids or less, preferably 10 amino acids or less, more preferably 8 amino acids or less, more preferably 5 amino acids or less, or more preferably 3 amino acids or less. When one or more amino acids are substituted, it is expected that the activity of the protein can be maintained by substitution with an amino acid having a side chain with the same property. Such substitution is referred to in this application as conservative substitution. Conservative substitution includes, for example, substitution between amino acids in any group including basic amino acid (e.g. lysine, arginine, histidine), acidic amino acid (e.g. aspartic acid, glutamic acid), uncharged non-polar amino acid (e.g, glycine, asparagine, glutamine, serine, threonine, tyrosine, cysteine), non-polar amino acid (e.g. alanine, valine, leucine, isoleucine, proline, phenylalanine, methionine, tryptophan), branched amino acid (e.g. threonine, valine, isoleucine) and aromatic amino acid (e.g. tyrosine, phenylalanine, tryptophan, histidine).

The modified protein has high homology to the amino acid sequence of the wild-type protein. For example, high homology is an amino acid sequence having an identity of 70% or greater, 75% or greater, 80% or greater, 85% or greater, 90% or greater, 93% or greater, 95% or greater, or 96% or greater. The identity of the amino acid sequence can be determined using, for example, a BLASTP program (Altschul, S. F. et al., J. Mol. Biol. 215: 403-410, 1990). For example, the NCBI (National Center for Biotechnology Information) BLAST web site allows performing a search using a default parameter. (Altschul S.F. et al., Nature Genet. 3:266-272, 1993; Madden, T.L. et al., Meth. Enzymol. 266:131-141, 1996; Altschul S.F. et al., Nucleic Acids Res.25:3389-3402, 1997; Zhang J. & Madden T.L., Genome Res. 7:649-656, 1997). For example, using the blast2sequences program (Tatiana A et al., FEMS Microbiol Lett. 174:247-250, 1999), which compares two sequences, an alignment of the two sequences can be made to determine the identity of the sequences. A gap is treated in the same way as a mismatch. For example, the identity value for the entire amino acid sequence of the natural type cytokine (a form of a mature type after secretion) is calculated. Specifically, the ratio of the number of matching amino acids to the total number of amino acids of the wild-type protein (mature type in the case of secretory protein) is calculated.

In addition, a gene or mRNA can include a silent mutation without changing the encoded amino acid sequence. In an AT(U)-rich gene, five or more consecutive bases of A or T(U) are substituted by G or C without changing the encoded amino acid sequence, thereby stably achieving high expression of the gene.

In another embodiment, a reprogramming factor may be a small molecule compound (e.g., a combination of VPA, CHIR99021, 616452, tranylcypromine, forskolin, and DZNep) used to induce dedifferentiation of differentiated cells, or miRNA. If the reprogramming factor is a small molecule, the reprogramming factor can be added to a medium (Science 09 Aug 2013: Vol. 341, Issue 6146, pp. 651-654).

### (Contact of Reprogramming Factor with Somatic Cells)

In step (2), the reprogramming factor is brought into contact with the somatic cells. When the reprogramming factor is a gene, the reprogramming factor can be brought into contact with the somatic cells by introducing an expression vector encoding the reprogramming factor into the somatic cells according to a known method. The type of expression vector is not particularly limited, and a known expression vector may be used. Examples of the expression vector include an episomal vector, an artificial chromosome vector, a plasmid vector and a viral vector.

The episomal vector is a vector that can replicate autonomously outside of a chromosome. Specific means of using the episomal vector are disclosed in Yu et al., Science, 324, 797-801 (2009). For example, an episomal vector can be used having loxP sequences arranged in the same direction on the 5' side and the 3' side of a vector element necessary for replication of the episomal vector. The episomal vector can be replicated autonomously outside a chromosome and thus can provide stable expression in a host cell without being integrated into a genome. However, it is desirable to remove the vector quickly after iPS cell production. The vector element required for replication of the episomal vector is held between two loxP sequences, and Cre recombinase is induced to act on the vector element to excise the vector element so that the autonomous replication function of the episomal vector can be eliminated. Thus, the vector can be removed from the iPS cells at an early stage.

Examples of the episomal vector include a vector containing a sequence necessary for autonomous replication derived from EBV or SV40 or the like as a vector element. In particular, the vector element necessary for autonomous replication is an origin of replication and a gene encoding a protein which is coupled to the origin of replication to control replication. For example, the vector element is a replication origin oriP and an EBNA-1 gene in EBV or a replication origin ori and an SV40LT gene in SV40.

Examples of artificial chromosome vectors include a YAC (yeast artificial chromosome) vector, a BAC (bacterial artificial chromosome) vector, a PAC (P1-derived artificial chromosome) vector, and a HAC (human artificial chromosome) vector.

The plasmid vector is not particularly limited if the plasmid vector can be expressed in somatic cells to be introduced. If the somatic cells to be introduced are mammalian, a normally used vector can be used as the plasmid vector for expressing animal cells. Examples of the plasmid vector for expressing animal cells include pA1-11, pXT1, pRc/CMV, pRc/RSV, and pcDNAI/Neo.

Examples of the viral vector include a retrovirus (including lentivirus) vector, an adenoviral vector, an adeno-associated viral vector, a Sendai viral vector, a herpesviral vector, a vaccinia viral vector, a poxviral vector, a polioviral vector, a Sindbis viral vector, a rhabdoviral vector, a paramyxoviral vector, and an orthomyxoviral vector. In the present specification, the viral vector means a vector having genomic nucleic acid derived from the virus and capable of expressing a transgene by integration of the transgene into the nucleic acid.

A Sendai viral vector is the preferred viral vector. A Sendai virus is a mononegaviral virus belonging to Paramyxoviridae (Paramyxoviridae; including genera such as Paramyxovirus, Morbillivirus, Rubulavirus and Pnemovirus) which contains a minus strand RNA (an antisense strand with respect to a sense strand encoding a virus protein) as a genome. The Sendai viral vector is a chromosome-nonintegrated viral vector, and the vector is expressed in the cytoplasm. Thus, the transgene is not integrated into a host chromosome, allowing the vector to be safely removed from the introduced cell once the cells are reprogrammed.

The Sendai viral vector includes, in addition to infectious virus particles, a composite of a virus core, a virus genome and a virus protein, or a composite containing non-infectious virus particles or the like and capable of expressing a gene to be integrated by introduction into cells. For example, a ribonucleoprotein (a core portion of the virus, i.e. RNP) composed of a Sendai virus genome and Sendai virus protein (NP, P and L protein) to be coupled to the genome can express the transgene in cells by introduction into the cells (WO00/70055). The introduction into the cells can be performed by a general method, such as electroporation, lipofection or microinjection. Such a ribonucleoprotein (RNP) is also included in the Sendai viral vector.

Examples of a known method for introducing an expression vector into somatic cells include an infection method for a viral vector (e.g., a retroviral vector (Cell 2007 Nov 30;131(5):861-72, a Sendai viral vector (Japanese Patent No. 5963309)) and a calcium phosphate method, a lipofection method, a RetroNectin method, or an electroporation method for a plasmid vector (Nat Methods.2011 May;8(5):409-12).

When the reprogramming factor is mRNA or miRNA, the contact of the reprogramming factor with somatic cells is, for example, a gene transfer method using synthetic mRNA (e.g., a calcium phosphate method, a lipofection method (Cell Stem Cell. 2010 Nov 5; 7(5): 618-630) or an electroporation method).

When the reprogramming factor is a protein, the reprogramming factor can be brought into contact with somatic cells, e.g., by a direct infusion method (e.g., a needle method, a lipofection method, or an electroporation method) of protein (e.g., a cell membrane permeable recombinant protein) (Cell Stem Cell, 4, May 8, 2009, 381-384).

Step (2) may be performed before or after step (1). In one embodiment, step (2) is performed after step (1). In another embodiment, step (2) is performed prior to step (1).

In one embodiment, the method of the present application includes the step of expanding culture of the somatic cells prior to step (2). The culture conditions may be appropriately determined by a person skilled in the art and are not particularly limited. For example, when the somatic cells are peripheral blood mononuclear cells (PBMC), CD34-positive cells may be expanded by culturing in CD34-positive cell culture medium. A known medium may be appropriately used as CD34-positive cell culture medium. For example, a culture medium containing IL-6, SCF, TPO, Flt-3L, IL-3, and G-CSF can be used.

The culture temperature is not limited, may be about 30 to 40°C, and for example, about 37°C. The culture is conducted in an atmosphere comprising CO₂, O₂, and N₂. The CO₂ concentration is about 0.05 to 15%, preferably about 3 to 7%, more preferably about 4 to 6%, and most preferably about 5%. The O₂ concentration is about 0.05 to 100% and preferably 2 to 25%. The N₂ concentration is about 0.05 to 100% and preferably 30 to 75%.

The period of expansion culture may be appropriately determined by a person skilled in the art and is not particularly limited. The period may be one to twenty days or three to ten days, for example four to seven days, during which the culture medium is replaced. In addition, the culture medium may be continuously replaced (perfusion culture) using, for example, an automatic culture apparatus.

In the expansion culture step, the somatic cells may be cultured by suspension culture. In the present application, "suspension culture" means that cells are cultured without adhering to a culture base material. Among commercially available cell culture containers, although not particularly limited, culture may be performed using a container in which no artificial treatment (e.g., coating with an extracellular matrix or the like) for improving adhesion with cells is performed or in which a treatment for artificially suppressing adhesion is performed (e.g., coating with polyhydroxyethyl methacrylate (poly-HEMA) or a polymer of 2-methacryloyloxyethyl phosphorylcholine (lipidure)). For example, commercial products such as 96-well low-adhesion plate (Sumitomo Bakelite Co., Ltd.) and 35-mm low-adhesion dish (Sumitomo Bakelite Co., Ltd.) may be used. If the somatic cells are floating somatic cells (e.g., blood cells such as peripheral blood mononuclear cells, hematopoietic progenitor cells, and CD34-positive cells), the floating somatic cells will not adhere to a culture vessel. Therefore, a culture container for adherent culture may also be used. For example, commercial products such as a 24F single well type adherent cell culture plate (Sumitomo Bakelite Co., Ltd.) may be used.

### - Step (3)

In step (3), the somatic cells in contact with the reprogramming factor are cultured in a state in which the two or more somatic cells are gathered in the compartment. The somatic cells may be cultured by suspension culture.

Step (3) is performed in a state where the two or more somatic cells are gathered in the compartment. For example, step (3) is performed in a state in which 2 to 500, 2 to 400, or 3 to 300 somatic cells may be gathered at a point in the compartment, and for example, 5 to 500, 10 to 500, 50 to 400, or 100 to 300 somatic cells, for example, about 200 somatic cells are gathered. If somatic cells are gathered in a linear portion in a compartment in step (3), the number of somatic cells to be gathered may be determined according to the length of the linear portion. In one embodiment, a theoretical value obtained by calculating the number of somatic cells accumulated on a cross section perpendicular to the linear portion is about one or more, about ten or more, about 100 or more, about 1,000 or more, or about 10,000 or more, for example, about one to 10,000 or about 100 to 1,000. Somatic cells may be gathered by actions such as rotating, rocking, and shaking the container, and somatic cells may be gathered at the bottom by using a compartment having an inclined surface extending from the opening to the bottom.

In step (3), the culture temperature is not limited, may be about 30 to 40°C, and for example, about 37°C. The culture is conducted in an atmosphere comprising CO₂, O₂, and N₂. The CO₂ concentration is about 0.05 to 15%, preferably about 3 to 7%, more preferably about 4 to 6%, and most preferably about 5%. The O₂ concentration is about 0.05 to 100% and preferably 2 to 25%. The N₂ concentration is about 0.05 to 100% and preferably 30 to 75%.

In step (3), the culture period of the somatic cells may be appropriately determined by a person skilled in the art based on, for example, the type of somatic cells, the type of reprogramming factor, the contact means of the reprogramming factor, and is not particularly limited. The culture period may be three to thirty days, five to twenty days, or eight to fifteen days, for example, about ten days. The generation of pluripotent stem cells can be confirmed by the expression of a pluripotent stem cell marker, e.g. SSEA-3, SSEA-4, TRA-1-60, TRA-1-81, TRA-2-49/6E, ALP, Sox2, E-cadherin, UTF-1, Oct3/4, Rexl and Nanog. Expression of pluripotent stem cell markers may be confirmed visually by immunostaining under a microscope, or by flow cytometry or fluorescence-activated cell sorting (FACS).

Produced pluripotent stem cells may be subcultured every three to thirty days, four to twenty days, or five to ten days, for example, every approximately seven days. The number of subculturing times is not particularly limited and may be, for example, one or more, two or more, three or more, four or more, five or more, ten or more, twenty or more, thirty or more, forty or more, fifty or more, sixty or more, seventy or more, eighty or more, ninety or more, or one hundred or more. The subculture period is not particularly limited and may be one day or more, ten days or more, twenty days or more, thirty days or more, forty days or more, fifty days or more, one hundred days or more, two hundred days or more, three hundred days or more, four hundred days or more, five hundred days or more, or 1000 days or more. For example, the container for suspension culture can be used as a container for subculture. When a closed automatic culture apparatus is used, subculture is not performed. The expansion and differentiation of the pluripotent stem cells may be performed by including the culture medium and/or refluxing the culture medium.

### (Gathering step)

In one embodiment, in the method of the present application, the compartment includes a gathering portion that can gather two or more somatic cells with low cell adhesion and the step of gathering two or more somatic cells is further included. For example, the two or more somatic cells can be gathered by at least one following action; rotating, rocking or shaking the container. When the method of the present application includes the gathering step, the step (3) is performed after two or more somatic cells are gathered in a compartment by the gathering step. In other words, two or more somatic cells in contact with the reprogramming factor can be cultured in a state where the two or more somatic cells are more securely gathered.

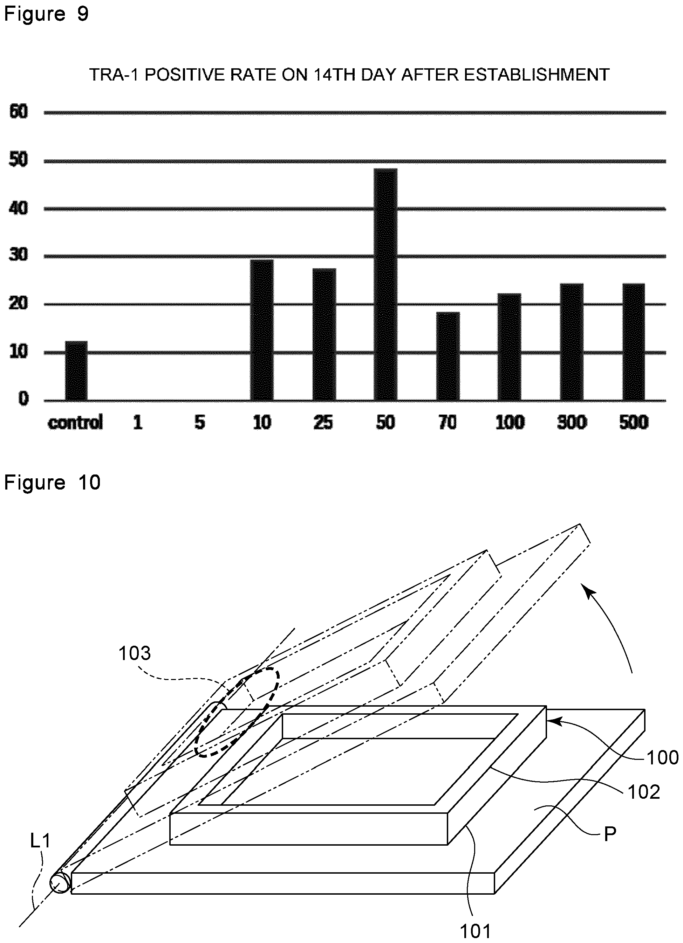

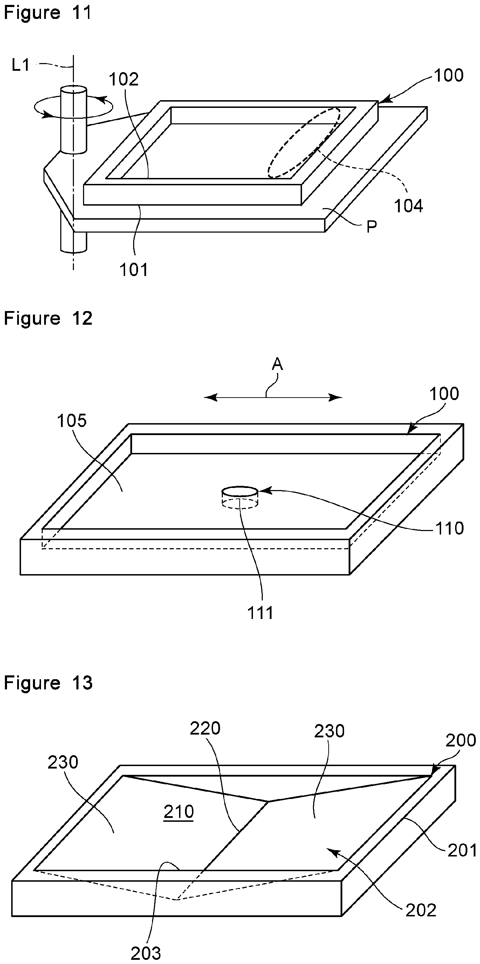

Referring to Figures 10 and 11, an example of the gathering step with a rotation on the container will be described below. A container 100 in Figures 10 and 11 is a dish substantially shaped like a rectangular solid including a compartment and has a plurality of surfaces including a first surface 101. The first surface 101 has the largest area among the plurality of surfaces of the container 100 and is opposed to an opening surface 102. The container 100 is rotated about a rotation axis L1, which is parallel to or crossing the first surface 101, while a plurality of somatic cells (not shown) are contained in the container 100, so that the plurality of somatic cells contained in the container 100 are gathered.

The container 100 of Figure 10 is configured to permit rotating about the rotation axis L1 parallel to the first surface 101. The plurality of somatic cells contained in the container 100 are gathered in a region 103 near the rotation axis L1 of the container 100. For example, assuming that the container 100 is placed on a horizontal surface P, when the container 100 is rotated above the horizontal surface P, the region 103 is located closest to the horizontal surface P at a side edge of the container 100. If both ends of the rotation axis L1 are not coupled to a bearing mechanism, but, for example, one end thereof is coupled to a bearing mechanism on the near side in Figure 10, and the bearing mechanism can tilt in the direction of an elevation angle while rotating the rotation axis L1 according to ball joint specifications or the like, the region 103 closest to the horizontal surface P is located at a corner of the container 100.

The container 100 of Figure 11 is configured to rotate about the rotation axis L1 crossing the first surface 101. A plurality of somatic cells contained in the container 100 are gathered in a region 104 remote from the rotation axis L1 of the container 100. For example, assuming that the container 100 is placed on the horizontal surface P, the container 100 rotated about the rotation axis L1 orthogonal to the horizontal surface P has the region 104 farthest from the rotation axis L1 at a side edge of the container 100.

With reference to Figure 12, an example of the gathering step by rocking or shaking the container is described below. The container 100 in Figure 12 is substantially in the form of a rectangular plate having a recessed portion 110. The recessed portion 110 has a substantially circular opening 111 on a surface 105 of the container 100. The container 100 is rocked or shaken in the direction of an arrow A of Figure 12 while a plurality of somatic cells are contained in the recessed portion 110, so that the plurality of somatic cells are gathered in the recessed portion 110.

The container 100 of Figures 10 and 11 may have recessed portions in the regions 103 and 104. The recessed portion has, for example, a bottom surface with low cell adhesion and is configured to permit gathering two or more somatic cells at the bottom. In addition, a plurality of recessed portions may be provided in the regions 103 and 104 according to the seeding amount of somatic cells. In this case, a required amount of somatic cells can be efficiently gathered.

A container of Figures 13 to 21, which will be described later, may be used for the gathering step of the present embodiment.

### [Container]

The present application provides a container comprising
a container body capable of containing somatic cells; and
at least one compartment disposed within the container body
wherein the compartment comprises
a gathering portion capable of gathering two or more somatic cells having low cell adhesion; and
a guiding portion that guides somatic cells that have flown into the container to the gathering portion. By using the container thus configured, two or more somatic cells can be gathering without rotating, rocking or shaking the container.

The container of the present disclosure may have at least one compartment. For example, the container may be in the form of a plate, a package, or a dish.

The container of the present disclosure may further comprise a passage portion which allows the inside of the container body and the outside of the container body to communicate with each other and allows somatic cells to pass therethrough.

The guide portion is not necessarily pre-configured in a shape and may be a passageway or means that allows somatic cells to be guided to the collection portion by an external force.

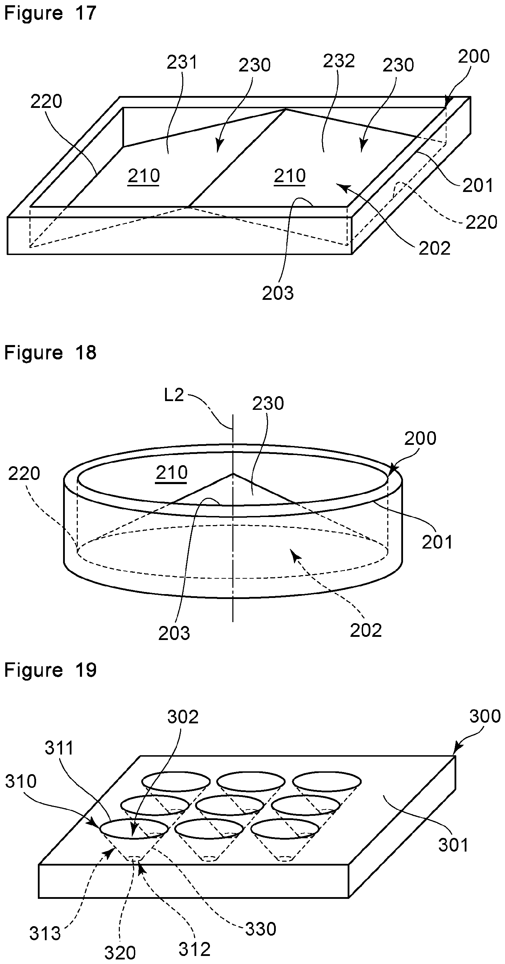

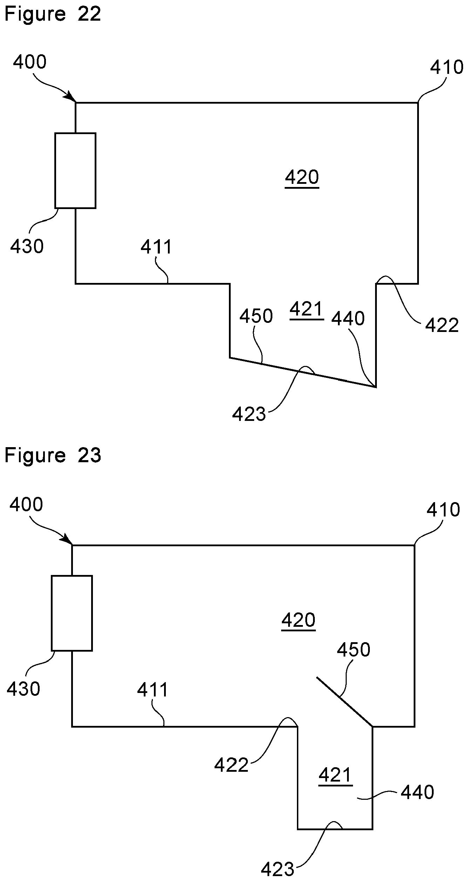

Figures 13 to 23 illustrate examples of the container of the present disclosure. Figures 13 to 18 illustrate examples of the dish-shaped container. Figures 19 to 21 illustrate examples of the plate-shaped container. Figures 22 and 23 illustrate examples of the package-shaped container.

A container 200 of Figures 13 to 17 is a substantially rectangular dish having at least one compartment 210. The compartment 210 has a gathering portion 220 and a guide portion 230. The container 200 has a substantially rectangular opening surface 201 having an opening 203 and a substantially rectangular bottom surface 202 opposed to the opening surface 201. The gathering portion 220 and the guide portion 230 are disposed on the bottom surface 202.

The container of Figure 13 includes the single compartment 210. In the container 200 of Figure 13, the gathering portion 220 is disposed substantially at the center of the bottom surface 202 in the longitudinal direction of the container 200 (hereinafter referred to as the longitudinal direction) and extends substantially linearly in the width direction of the container 200 (hereinafter referred to as the width direction). The bottom surface 202 of the container 200 is configured with two inclined surfaces extending from both ends in the longitudinal direction to the center in the longitudinal direction. The two inclined surfaces are inclined from the opening surface 201 to the bottom surface 202 as extending from both ends in the longitudinal direction to the center in the longitudinal direction and constitute the guide portion 230. In the container 200 of Figure 13, somatic cells that have flown into the container 200 are linearly gathered by the plurality of inclined surfaces configured with flat surfaces. An angle formed by the inclined surfaces constituting the guide portion 230 and a surface (e.g., the opening surface 201) of the container 200 is preferably 15° to 60°.

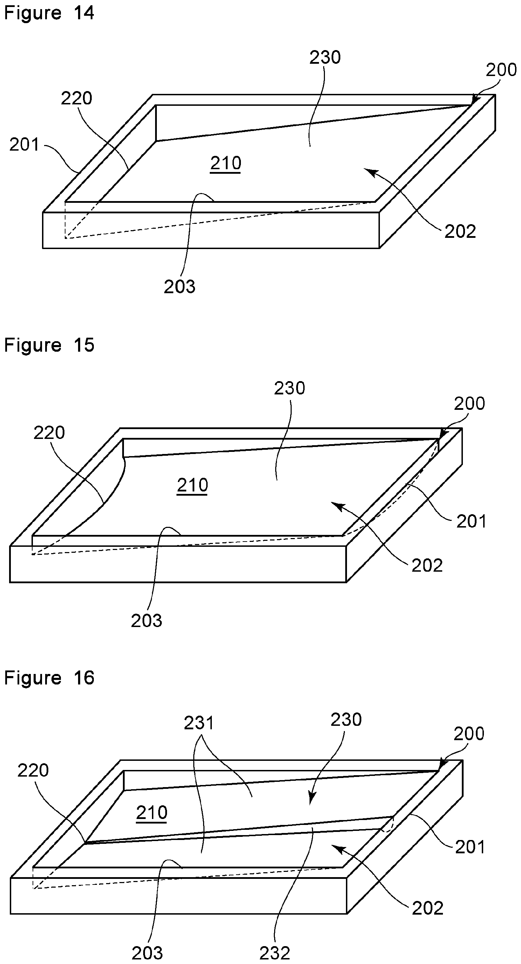

The container of Figure 14 includes the single compartment 210. In the container 200 of Figure 14, the gathering portion 220 is disposed at one end in the longitudinal direction of the bottom surface 202 and extends substantially linearly in the width direction. The bottom surface 202 of the container 200 is configured with an inclined surface extending from the other end in the longitudinal direction to one end in the longitudinal direction. The inclined surface slopes from the opening surface 201 to the bottom surface 202 as it extends from the other end in the longitudinal direction to one end in the longitudinal direction and constitutes the guide portion 230. In the container 200 of Figure 14, somatic cells that have flown into the container 200 are linearly gathered by the plurality of inclined surfaces configured with flat surfaces. An angle formed by the inclined surface constituting the guide portion 230 and a surface (e.g., the opening surface 201) of the container 200 is preferably 15° to 60°.

The container 200 of Figure 15 differs from the container 200 of Figure 14 in that the inclined surface constituting the guide portion 230 is configured with a curved surface protruding in a direction extending from the opening surface 201 to the bottom surface 202. In the container 200 of Figure 15, somatic cells that have flown into the container 200 are linearly gathered by the inclined surface configured with a curved surface.

The container 200 of Figure 16 includes the single compartment 210. In the container 200 of Figure 16, the gathering portion 220 is a point that is located at one end in the longitudinal direction of the bottom surface 202 and substantially at the center in the width direction. The bottom surface 202 of the container 200 is configured with an inclined surface 231 extending from the other end in the longitudinal direction to the gathering portion 220 and a groove portion 232 disposed substantially at the center of a first inclined surface 231 in the width direction. The inclined surface 231 and the groove portion 232 constitute the guide portion 230. The inclined surface 231 is inclined from the opening surface 201 to the bottom surface 202 as extending from the other end in the longitudinal direction to one end in the longitudinal direction and tilts from the opening surface 201 to the bottom surface 202 while extending from both ends in the width direction to the center in the width direction. The groove portion 232 is opened at the inclined surface 231 and has a groove width (in other words, the groove width of the groove portion 232 in the width direction) that decreases from the other end in the longitudinal direction to one end in the longitudinal direction. In the container 200 of Figure 16, somatic cells that have flown into the container 200 are gathered to a point by the plurality of inclined surfaces configured with flat surfaces. The groove portion 232 may be omitted.

The container 200 of Figure 17 includes two compartments 210 disposed side by side in the longitudinal direction. In the container 200 of Figure 17, the gathering portion 220 of each of the compartments 210 is disposed at one end in the longitudinal direction of the bottom surface 202 and extends substantially linearly in the width direction. The bottom surface 202 of the container 200 is configured with a first inclined surface 231 extending substantially from the center in the longitudinal direction to one end in the longitudinal direction and a second inclined surface 232 extending substantially from the center in the longitudinal direction to the other end in the longitudinal direction. The first inclined surface 231 and the second inclined surface 232 form the guide portion 230 of each of the compartments 210. The first inclined surface 231 and the second inclined surface 232 incline from the opening surface 201 to the bottom surface 202 as they extend from the center in the longitudinal direction to one end or the other end in the longitudinal direction. In the container 200 of Figure 17, somatic cells that have flown into the container 200 are guided to one of the two gathering portions 220 and are linearly gathered by the two inclined surfaces configured with flat surfaces. An angle formed by the first inclined surface 231 and the second inclined surface 232 and a surface (e.g., the opening surface 201) of the container 200 is preferably 15° to 60°.

The container 200 of Figure 18 includes the single compartment 210. The container 200 of Figure 18 is a substantially circular dish having a substantially circular opening surface 201 and a substantially circular bottom surface 202. The gathering portion 220 is disposed at one end of the bottom surface 202, remote from a center line L2 in a radial direction and extends linearly in a circular shape. The bottom surface 202 of the container 200 is substantially conical in shape with a vertex disposed on the center line L2 that passes through the center of the container 200 and is orthogonal to the opening surface 201 and the bottom surface 202, and is configured with an inclined surface extending from the center line L2 in a radial direction. The inclined surface slopes from the opening surface 201 to the bottom surface 202 as it separates from the center line L2 and constitutes the guide portion 230. In the container 200 of Figure 18, somatic cells that have flown into the container 200 are linearly gathered in a circular shape by the inclined surface configured with a curved surface. An angle formed by the first inclined surface 231 and the second inclined surface 232 and a surface (e.g., the bottom surface 202) of the container 200 is preferably 15° to 60°.

Figures 13 to 18 illustrate examples in which an inclination or a slope is preformed on the bottom portion of the container. An inclination or a slope may be provided at the bottom of the container by forming the bottom portion of the container by using, for example, a flexible member made of a silicon material or the like. In this case, for example, a tilt mechanism is provided under the bottom of the container under MEMS (Micro Electro Mechanical Systems) control, and the tilt mechanism is activated to form an inclination or a slope at the bottom of the container. As another aspect, a member having a surface corresponding to the inclination or slope in Figures 13 to 18 may be provided under the bottom formed by a flexible member and may be pressed vertically from the bottom to the top to form a predetermined inclination or slope in each container.

A container 300 of Figure 19 includes nine compartments 302. Each of the compartments 302 includes a recessed portion 310. The container 300 of Figure 19 is substantially shaped like a rectangular plate having an opening surface 301 with the nine recessed portions 310. The recessed portion 310 has an opening 311 formed on the opening surface 301 and a bottom surface 312 opposed to the opening 311. The recessed portion 310 is substantially shaped like a truncated cone tapering from the opening 311 to the bottom surface 312. The bottom surface 312 constitutes a gathering portion 320, and a side surface 313 connected to the opening 311 and the bottom surface 312 forms a guide portion 330. In the container 200 of Figure 19, somatic cells that have flown into the recessed portion 310 are gathered linearly to a location by the inclined surface configured with a curved surface. An angle formed by the side of the recessed portion 310 constituting the guide portion 330 and a surface (e.g., the opening surface 301) of the container 200 is preferably 15° to 60°.

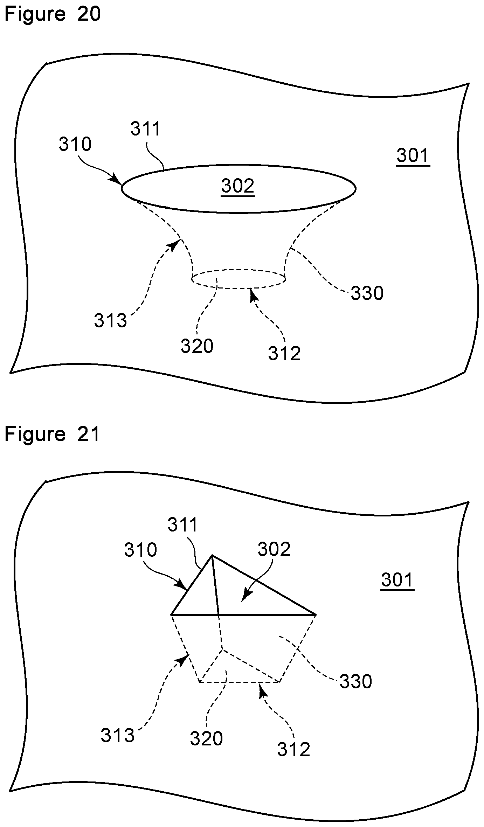

For the recessed portion 310, any configuration that is capable of gathering two or more somatic cells that flow into the recessed portion 310 may be used. For example, as illustrated in Figure 20, the recessed portion 310 may be substantially shaped like a truncated cone with the curved side surface 313 that protrudes toward the center of the recessed portion 310, or may be substantially shaped like a prismoid as shown in Figure 21. In the recessed portion 310 of Figure 20, somatic cells that have flown into the recessed portion 310 are gathered at one location by the inclined surface configured with a curved surface as in Figure 19. In the recessed portion 310 of Figure 21, somatic cells that have flown into the recessed portion 310 are gathered at a location by the inclined surface configured with a polygonal (e.g., substantially triangular) surface.

In Figures 10 to 21, the opening surface need not have an opening over the entire surface. An opening may be formed on a part of a wall surface, and the opening may be opened or closed by a cap mechanism or the like. The opening surface need not be opposed to the bottom surface and may be provided on the side of the container as long as the idea of the invention is shared.

A container 400 of Figure 22 includes a container body 410 that can contain somatic cells, at least one compartment 420, and a passage portion 430 that allows the passage of somatic cells. The container body 410 is packaged with the compartment 420 disposed therein. The passage portion 430 allows the inside of the container body 410 and the outside of the container body 410 to communicate with each other. The compartment 420 includes a gathering portion 440 capable of gathering two or more somatic cells with low cell adhesion, and a guide portion 450 that guides somatic cells that have flown into the container 400 to the gathering portion 440. In Figure 22, the compartment 420 includes a recessed portion 421. The recessed portion 421 has an opening 422 formed on an inner surface 411 of the container body 410 and a bottom surface 423 that has low cell adhesion and is opposed to the opening 422. The bottom surface 423 includes a gathering portion 440 disposed at one end in the longitudinal direction, and an inclined surface that tilts from the opening 422 to the bottom surface 423 as extending from the other end in the longitudinal direction to one end in the longitudinal direction. The inclined surface constitutes a guide portion 450. For the recessed portion 421, any configuration capable of gathering two or more somatic cells having flowing into the container body 410 can be used. For example, the recessed portion 310 of Figures 19 to 21 may be used as the recessed portion 421.

The container 400 of Figure 23 differs from the container 400 of Figure 22 in that the gathering portion 440 is configured with the recessed portion 421 and the guide portion 450 is configured with a protruding portion that protrudes from the inner surface 411 of the container body 410. The recessed portion 421 has the bottom surface 423, which has low cell adhesion and is substantially parallel to the inner surface 411 of the container body 410. The protruding portion constituting the guide portion 450 is adjacent to the opening 422 outside the recessed portion 421, and is located more distant from the passage portion 430 than the opening 422. Thus, the guide portion 450 is not always a part of the recessed portion 421.

The passage portion 430 may be configured to perform all of the supply and discharge of somatic cells and the supply of reprogramming factors. Further, the passage portion 430 may be configured with a first passage portion that supplies and discharges somatic cells and a second passage portion that supplies the reprogramming factor or may be configured with a first passage portion that supplies somatic cells, a second passage portion that discharges somatic cells, and a third passage portion that supplies the reprogramming factor.

The shape and configuration of the recessed portion 421 of the container 400 are not limited to those of Figures 22 and 23. For example, the container 400 may be identical in shape and configuration to the containers 100, 200, and 300 shown in Figures 12 to 21.

The container of the present disclosure may contain the reprogramming factor in the compartment. The reprogramming factor may be used as the reprogramming factor in the compartment or may be the protein, a small molecule compound, or miRNA. The amount of the reprogramming factor contained in the compartment may be appropriately determined by a person skilled in the art and is not particularly limited.

### [System]

The present application provides a system comprising:
the container;
a seeding device that supplies somatic cells into the container and seeds the somatic cells;
a collecting device that discharges somatic cells in contact with a reprogramming factor from the inside of the container to the outside of the container and collects the somatic cells; and
a controller that controls the seeding device and the collecting device. With this system, pluripotent stem cells can be produced from somatic cells.

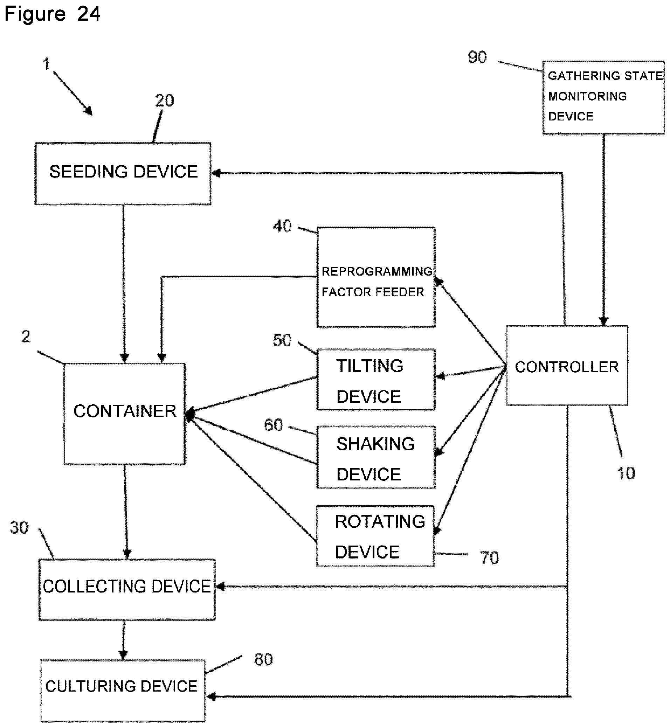

As illustrated in Figure 24, a system 1 of the present disclosure includes a container 2, a controller 10, a seeding device 20, and a collecting device 30. As an example, the system 1 of Figure 24 further includes a reprogramming factor feeder 40, a tilting device 50, a shaking device 60, a rotating device 70, and a culturing device 80.

The container 2 may be a container having one or more compartments with low cell adhesion. For example, the containers 100, 200, 300, and 400 in Figures 10 to 23 may be used.

The controller 10 includes, for example, a CPU that performs operations and a memory device that stores programs necessary for the operations and results of the operations. In the system 1 according to the present embodiment, the controller 10 controls the reprogramming factor feeder 40, the tilting device 50, the shaking device 60, the rotating device 70, and the culturing device 80, in addition to the seeding device 20 and the collecting device 30, so that pluripotent stem cells are produced from somatic cells. The controller 10 monitors the operating conditions of various devices electrically connected to the controller 10. For example, when the container 100 in Figure 10 is used as the container 2, a control signal for a tilt angle is transmitted to the tilting device 50 at any time after seeding of somatic cells. Upon receiving the control signal from the controller 10, the tilting device 50 tilts the first surface 101 of the container 100 according to the tilt angle. Thus, a guided state like the guide portion 230 of the container 200 in Figure 14 can be created in the container 100. The tilting device 50 desirably has the function of adjusting a tilting angle in a plurality of steps or without steps, thereby controlling a gathering state of somatic cells more precisely than in the case where a guide portion is provided in advance in a container. When the container 100 in Figure 11 is used, the controller 10 transmits, to the rotating device 70, control signals for a rotation angle, a rotation speed, an rpm, an acceleration, an angular velocity, and a torque or the like of the rotation axis L1 of the container 100 to the rotating device 70 at any time after seeding of somatic cells. When receiving the control signal from the controller 10, the rotating device 70 gathers somatic cells in the region 104 in the container 100 according to a centrifugal force based on the rotation. In this case, when the container 100 is made of an optically visible material, a gathering-state monitoring device 90 monitors a gathering state of somatic cells of the container 100 and outputs the result as a monitor signal to the controller 10. The controller 10 can sequentially receive the monitor signals from the gathering-state monitoring device 90 and generate an optimal rotation on the container 100 by inverter control according to the gathering state of somatic cells. Thus, pluripotent stem cells can be produced without applying an excessive stress to the somatic cells and the pluripotent stem cells. In addition, a control mechanism configured with a combination of the controller 10 and the tilting device 50 or the rotating device 70 can be effectively used even when the reprogramming factor is brought into contact with somatic cells.

The seeding device 20 supplies somatic cells to the container 2 before the somatic cells are brought into contact with the reprogramming factor, so that the somatic cells are seeded in the container 2. In the present embodiment, the seeding device 20 includes, for example, a storage tank in which somatic cells are stored before being brought into contact with the reprogramming factor, and a drive unit (e.g., a pump) that delivers somatic cells in the storage tank to the container 2.

The collecting device 30 discharges somatic cells in contact with the reprogramming factor from the inside of the container 2 to the outside of the container 2 and collects the somatic cells. In the present embodiment, the collecting device 30 includes, for example, a drive unit that discharges somatic cells in contact with the reprogramming factor from the inside of the container 2 to the outside of the container 2, and a storage tank that temporarily stores somatic cells discharged by the drive unit. Somatic cells stored in the storage tank are delivered by the drive unit to the culturing device 80.

The reprogramming factor feeder 40 comprises, for example, a storage tank in which the reprogramming factor is stored, and a drive unit which delivers the reprogramming factor in the storage tank to the container 2.

The tilting device 50 is configured to tilt a part of the container 2 or the entire container 2 with respect to a reference surface (e.g., the horizontal surface P in Figures 10 and 11) so as to cause two or more somatic cells in the container 2 to gather in the compartment of the container 2. For example, as shown in Figure 10, the tilting device 50 is configured to rotate and tilt the container 2 about the rotation axis L1 parallel to the reference surface or to deform a portion of the bottom of the container 2 if the container 2 has a flexible bottom.

The shaking device 60 is configured to rock or shake the container 2 so as to cause the two or more somatic cells in the container 2 to gather in the compartment of the container 2.

The rotating device 70 is configured to rotate the container 2 about the rotation axis L1 so as to cause the two or more somatic cells in the container to gather in the compartment by a centrifugal force.

The culturing device 80 cultures somatic cells in contact with the reprogramming factor, the somatic cells being collected by the collecting device 30.

The gathering-state monitoring device 90 includes, for example, an optical member such as a CCD (charge-coupled device) provided on a part of the rotation axis L1 or near the orbit of the container 2. The gathering-state monitoring device 90 is configured to monitor a gathering state of somatic cells when the container 100 is made of an optically visible material.

The system 1 may comprise five drive units for driving the seeding device 20, the collecting device 30, the reprogramming factor feeder 40, the tilting device 50, and the shaking device 60, respectively, or may comprise one to four drive units for driving some or all of the seeding device 20, the collecting device 30, the reprogramming factor feeder 40, the tilting device 50, and the shaking device 60.

The system of the present application may comprise an automated system for producing pluripotent stem cells from somatic cells. In this case, in addition to the foregoing devices, a device for more efficiently obtaining the automation may also be provided. When pluripotent stem cells are produced in accordance with GMP (Good Manufacturing Practice) standards, all of the devices constituting the system of the present application are preferably housed as an integrated system in a same dust-proof enclosure. In this case, however, the system of the present application may be configured to share some of the steps with other devices or allow an operator of the system of the present application to perform some of the steps. The devices constituting the system of the present application need not all be installed in the same facility. For example, only the controller 10 may be installed in a different facility and remotely connected to various devices such as the seeding device via the Internet.

The reprogramming factor feeder 40, the tilting device 50, the shaking device 60, the rotating device 70, the culturing device 80, and the gathering-state monitoring device 90 may be omitted. If the reprogramming factor feeder 40 is omitted, for example, the container 2 may be configured to include the reprogramming factor in the compartment in advance. If the tilting device 50 and the shaking device 60 are omitted, for example, the containers 200, 300, and 400 in Figures 13 to 23 may be used.

### [Kit]

The present application provides a kit for producing pluripotent stem cells from somatic cells, the kit comprising
a container with low cell adhesion, wherein the container has one or more compartments each capable of gathering two or more somatic cells; and
a reprogramming factor. In this case, the kit may be configured such that the reprogramming factor is sealed in advance in the container or is sealed in another ampoule or the like. When a kit having the former configuration is provided, a kit user does not need to introduce another reprogramming factor after seeding somatic cells, and the somatic cells can be reprogrammed only by seeding the somatic cells in the container. Thus, pluripotent stem cells can be more easily produced under axenic conditions.

Examples of the container and the reprogramming factor that are used in the present aspect are configured as described above. The kit of the present application may further include a buffer solution, a culture medium, and an instruction manual or the like.

### [Program]

The present application further provides a program for causing a computer to perform the method for producing pluripotent stem cells from somatic cells that is disclosed in the present application. For example, the method for producing pluripotent stem cells from somatic cells is implemented by executing a predetermined program by the CPU of the controller 10 in the system 1 of Figure 24.

The program may be stored and delivered to the computer using various types of non-transitory computer-readable media. Non-transitory computer-readable media include, for example, a magnetic recording medium (e.g., a flexible disk, a magnetic tape, or a hard disk drive), a magneto-optical recording medium (e.g., a magneto-optical disk), a CD-ROM, a CD-R, a CD-R/W, and a semiconductor memory (e.g., a mask ROM, a PROM, a flash ROM, or a RAM). In addition, the program may be delivered to the computer over wired communication channels, such as an electric wire and an optical fiber or over a radio channel.

### [Method For Producing Differentiated Cells]

The present application provides a method for producing differentiated cells, the method comprising the steps of:
providing pluripotent stem cells produced by the method of the present application; and
culturing the provided cells in a culture medium for inducing cell differentiation.

The differentiated cells are not particularly limited but include muscular system cells such as a cardiac muscle cell and a skeletal myoblast, nervous system cells such as a neuron, oligodendrocyte, and a dopamine-producing cell, retinal cells such as a retinal pigment epithelial cell, hematopoietic system cells such as a blood cell and a myelocyte, immune cells such as a T cell, an NK cell, an NKT cell, a dendritic cell, and a B cell, cells constituting organs such as a hepatocyte, a pancreatic beta cell, and a nephrocyte, a chondrocyte, and gem cells or the like, and a progenitor cell or a somatic stem cell (e.g., a mesenchymal stem cell, a hematopoietic stem cell, and a neural stem cell) that is differentiated into these cells. In one embodiment, a differentiated cell is a cardiac muscle cell.

Induction of differentiation of pluripotent stem cells can be performed by any known technique. The conditions for differentiation are not particularly limited and may be appropriately adjusted according to the target differentiated cells. For example, the differentiation may be carried out by culturing pluripotent stem cells in a culture medium for inducing cell differentiation, wherein the culture medium for inducing cell differentiation is prepared by adding a predetermined cytokine, a growth factor, or other compounds of a predetermined concentration to a culture fluid. When the differentiated cells are cardiomyocytes, pluripotent stem cells may be subjected to induction of differentiation into cardiac muscle cells according to a method described in WO2021/172542.

### EXAMPLES

The present application will be described in more detail below using examples. The present application is not limited by the examples. In the following examples, a day on which a reprogramming factor is brought into contact with somatic cells (if the contact lasts for several days, the starting date of the contact) is referred to as day 0, and the number of days that have elapsed is referred to as day X. Thus, for example, day 2 indicates two days after a day on which a reprogramming factor is brought into contact with somatic cells.

### [Example 1]

### 1. Method of Implementation

### 1-1. Used Reagent

Table 1 shows the detail of reagent used in the present example.

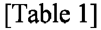

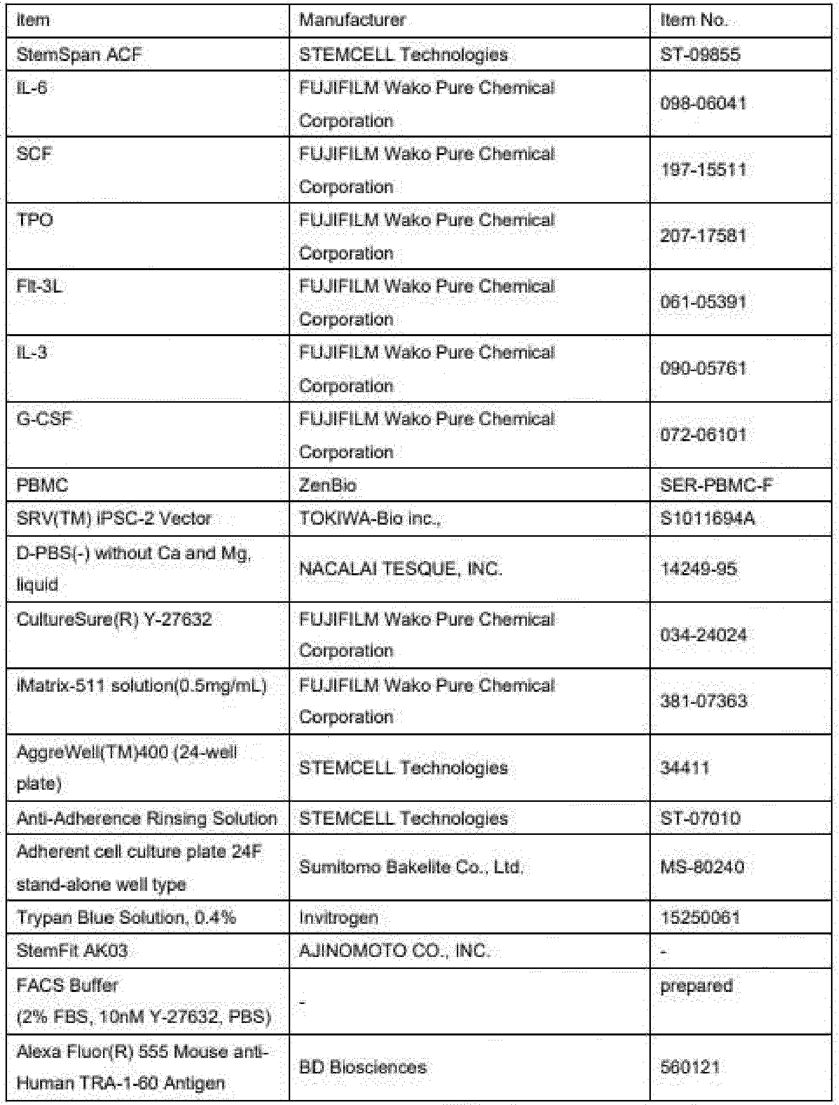

A SRV(TM) iPSC-2 vector is a stealth RNA vector with a gene expression level optimized to produce iPS cells from a peripheral blood monocyte, a peripheral blood mononuclear cell, and a CD34-positive cell, and carries four human-derived reprogramming genes: a human OCT3/4 gene, a human KLF4 gene, a human SOX2 gene, and a human c-MYC gene, an EGFP gene derived from crystal jelly, and a puromycin (Puro) resistance gene derived from Streptomyces alboniger. The introduction of the SRV(TM) iPSC-2 vector into cells can be confirmed by observing fluorescence of EGFP under a fluorescence microscope. SRV(TM) iPSC-2 Vector is automatically deleted in response to the expression of miR-302, a micro RNA expressed specifically in pluripotent stem cells. SRV(TM) iPSC-4 Vector may be used.

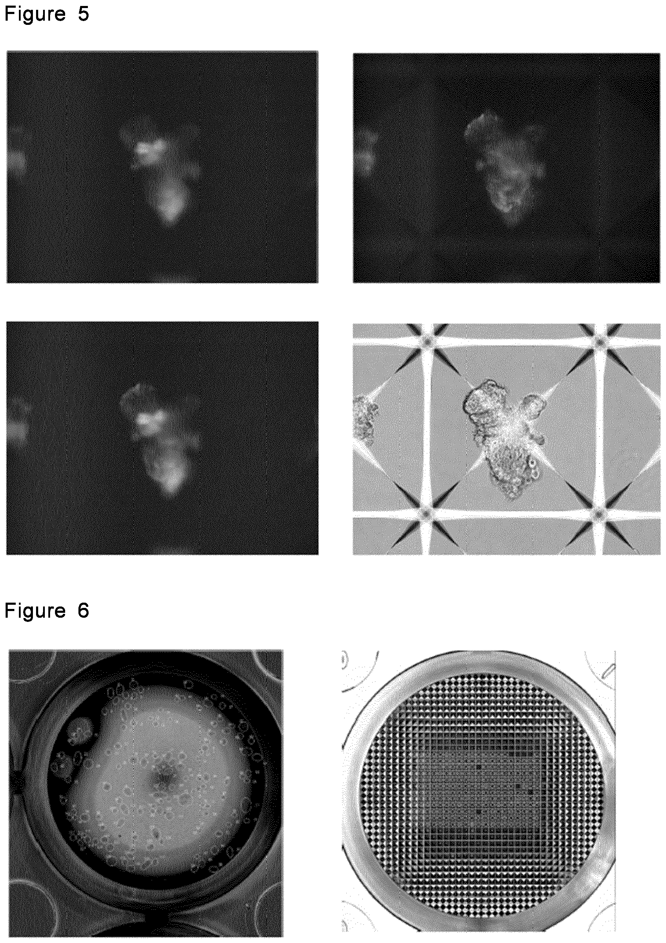

AggreWell(TM) plate has 1200 microwells with a diameter of 400 µm in a well (see the right image in Figure 6). Cells are seeded on AggreWell(TM) plate and are cultured therein, thereby producing spheroids of equal size.

### 1.2 Used Equipment

Table 2 shows the detail of equipment used in the present example.

**[Table 2]**

| Equipment | Model No. | Manufacturer |
|---|---|---|
| CO2 incubator | 370A | Thermo Fisher Scientific |
| Automatic cell counter Countess II | - | Thermo Fisher Scientific |
| Inverted microscope | CKX41 | Olympus Corporation |
| Fluorescence microscope | BZ-X810 | Keyence Corporation |
| Low speed refrigerated centrifuge | AX-511 | TOMY SEIKO CO., LTD. |

### 2. Origin cell

Table 3 shows the detail of cells (PBMC, ZenBio) used in the present example. Frozen PBMC was defrosted and used for the present example.

**[Table 3]**

| Name | Sex | Age | Race |
|---|---|---|---|
| PBMC | Female | 38 | Native Indian |

### 3. Procedure

### A. Expansion culture of CD34-positive cells

A1: 2 µL of cytokine adjusted to the concentrations in table 4 was added to 2 mL of StemSpan ACF to prepare CD34-positive cell culture medium.

**[Table 4]**

| | stock concentration (µg/mL) |
|---|---|
| IL-6 | 50 |
| SCF | 50 |
| TPO | 10 |
| Flt-3L | 20 |
| IL-3 | 20 |
| G-CSF | 10 |

A2: 1 mL of PBS for moisture retention was added to the outer wells of a 24-well plate.

A3: PBMC were thawed slightly in a water bath at 37°C, and approximately 1 mL of CD34-positive cell culture medium was added and was completely melted.

A4: Centrifugation was performed at 1500 rpm for five minutes.

A5: The supernatant was removed, and the cell pellets were disentangled by tapping and then were suspended by 1 mL of CD34-positive cell culture medium.

A6: A cell suspension was seeded on the 24-well plate and was subjected to stationary culture in a 5% CO2 incubator at 37°C for five days.

### B. Production of iPS cells (adherent culture: control)

B1: 5 mL of CD34-positive cell culture medium was prepared according to step A1. B2: The total amount of cultured PBMC was collected in a 1.5-mL tube, and the cells were counted. The result is shown in Table 5.

**[Table 5]**

| | PBMC |
|---|---|
| Total( × 10^5cells/mL) | 14.9 |
| Live( × 10^5cells/mL) | 10.7 |
| Dead( × 10^5cells/mL) | 4.22 |
| Viability(%) | 72 |

B3: An aliquot of 5×10⁵ cells (467 µL) was split and centrifuged with 300 G at 4°C for five minutes.

B4: The amount of vector was calculated to obtain a MOI = 3(TITER 4.3×10⁷CIU/mL, 150×10⁴/4.3×10⁷ = 34.88(µL))

B5: 34.88 µL of vector and 965.12 µL of CD34-positive cell culture medium were mixed to prepare a total amount of 1 mL of vector solution.

B6: The supernatant was removed from the centrifuged cell suspension, the cell pellets were disentangled by tapping and then were suspended in 1 mL of vector solution, and the cells were then brought into contact with the reprogramming factor (= Day 0). The cell density at infection was 0.5×10⁶ cells/mL.

B7: Incubation was performed in a 5% CO2 incubator at 37°C for 2 hours. The tube was gently tapped every 30 minutes.

B8: The cell suspension was transferred to a 15-mL tube, 1 mL of StemSpan ACF was added to the suspension, and then the suspension was centrifuged at 300 G at 4°C for five minutes.

B9: The supernatant was removed, and cell pellets were disentangled by tapping. B10: Steps B8 and B9 were repeated.

B11: 400 µL of cell CD34-positive cell culture medium and 1.8 µL of iMatrix were added to the 24-well plate and then the plate was agitated by shaking.

B12: Cells were seeded at 2×10⁴ cells/well (40 µL).

B13: The plate was placed in a 5% CO2 incubator at 37°C, and then the culture was started.

B14: 400 µL of an AK03 culture medium was added on Day 2 and Day 4.

B15: 400 µL of the AK03 culture medium was replaced on Day 7 and Day 9.

B16: The culture was transferred to a 6-well plate on Day 10.

### C. Production of iPS cells (suspension culture: AggreWell(TM) plate)

C1: 2 mL of Anti-Adherence Rinsing Solution was added to one well of an AggreWell(TM) plate to remove air bubbles in a microwell and then was centrifuged at 2000 G for five minutes.

C2: Rinsing Solution was removed by aspirator, and 2 mL of StemSpanACF was added and then removed by aspirator.

C3: 500 µL of CD34-positive cell culture medium was added to each well and then placed int the incubator.

C4: An aliquot of 1.2×10⁵ cells was split from the cell suspension (240µL) obtained in step B10.

C5: 760 µL of CD34-positive cell culture medium was added to the cell suspension so that the total amount of culture medium in one well was 1.5 mL.

C6: The AggreWell(TM) plate was removed from the incubator, and the whole amount was seeded.

C7: Pipetting was performed several times to evenly distribute the cells in the well.

C8: Centrifugation was performed at 100 G for three minutes to settle the cells in the microwell.

C9: The culture medium was put in the 5% CO2 incubator at 37°C.

C10: The culture medium was exchanged on Day 2, Day 4, Day 7 and Day 9. The exchange was performed by removing 750µL of the culture medium and adding 750µL of an AK03 culture medium.

C11: Immunostaining was performed with TRA-1-60 on Day 10 and was observed under a fluorescence microscope (the method will be described in step D).

C12: The culture was transferred to an AggreWell(TM) plate on Day 11.

### D. Immunostaining (TRA-1-60)

D1: 2 µL of a TRA-1-60 antibody was added to 100 µL of FACS buffer and was mixed therein.

D2: To prevent aspiration of a sphere, the culture medium was removed with a micropipette, leaving the culture medium with at a height of approximately 5 mm from the bottom surface of the well.

D3: 1 mL of FACS Buffer was added gradually from the edge of the well.

D4: FACS buffer was removed as in step D2.

D5: 100µL of the TRA-1-60 antibody diluted as in step D1 was added.

D6: Light was blocked with aluminum foil, and incubation was performed at room temperature for 30 minutes.

D7: 1 mL of FACS buffer was added, and then the culture medium was removed with a micropipette while leaving the culture medium at a height of approximately 5 mm from the bottom surface of the well.

D8: Step D7 was repeated.

D9: 300 µL of FACS buffer was added.

D10: Observation was performed under a Keyence fluorescence microscope.

### 4. Results

### Cell Morphology

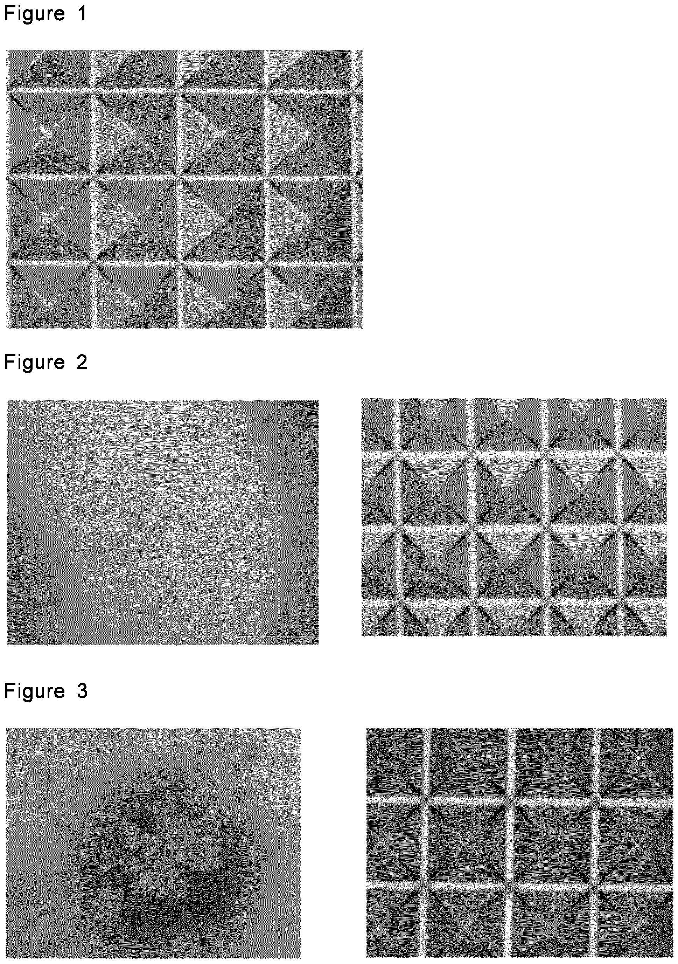

Immediately after seeding, cell colonies gathered at the bottom surface of the microwell of AggreWell(TM) were confirmed (Figure 1). On Day 4, spheroids of some cells were confirmed at the bottom of each microwell of an AggreWell(TM) plate (the right image in Figure 2). On Day 10, multiple spheroids of cells are formed, instead of round spheres, in the microwells of the AggreWell(TM) plate (the right image in Figure 3). In the right image in Figure 3, spheroids of cells were confirmed in all 12 microwells in a field of view. Multiple colonies were confirmed in adherent culture of control as well (the left image in Figure 6).

### TRA-1-60-positive cells

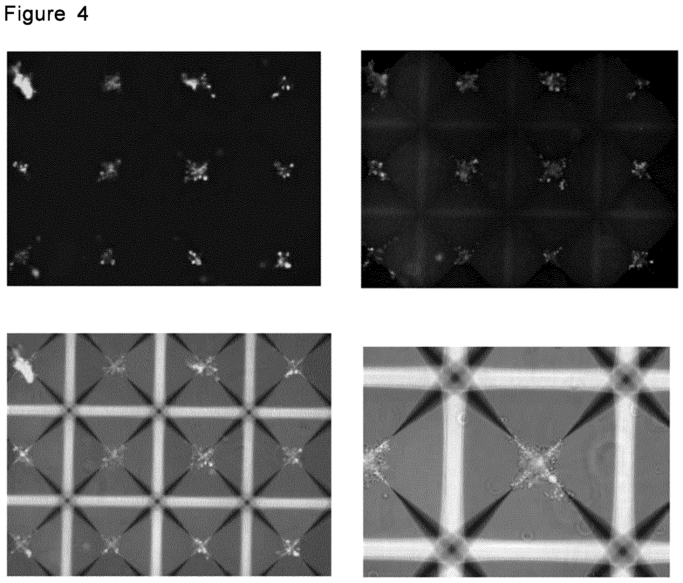

Expression of TRA-1-60, a pluripotent stem cell marker, was confirmed by immunostaining with TRA-1-60 antibody. On Day 10, TRA-1-60-positive cells were confirmed in almost all of the formed spheroids (the upper right image in Figure 4). Thus, it was found that iPS cells could be generated in a suspended state using an AggreWell(TM) plate. In addition, GFP-positive vector remaining cells were also confirmed in the same spheroids (the upper left image in Figure 4). This suggests that the cells were chimeric spheroids of transformed iPS cells and cells that were in the process of transformation. This state was also maintained in spheroids subcultured once (Figure 5).

### Summary

The result of example 1 is shown in Table 6. As shown in Table 6, PBMCs were aggregated at the beginning of the reprogramming step to efficiently obtain iPS cells.

**[Table 6]**

| Culture conditions | Number of iPS cell colonies | Establishment efficiency |
|---|---|---|
| Conventional method: cell-scaffolod dependent culture (iMatrix coated 24well plate) | 132 | 1.1% |
| Suspension culture 24well Aggrewell(TM)plate | 1145 | 9.5% |

| | | |
|---|---|---|
| Number of seeded cells: 1.2×10⁴ cells/well Reprograming period: 9 days Reprograming reagent: TOKIWA-Bio inc., SRV(TM)-iPSC-2 vector | | |

### [Example 2]

### Relationship between the size of a spheroid formed first (the number of constituent cells) and reprogramming efficiency

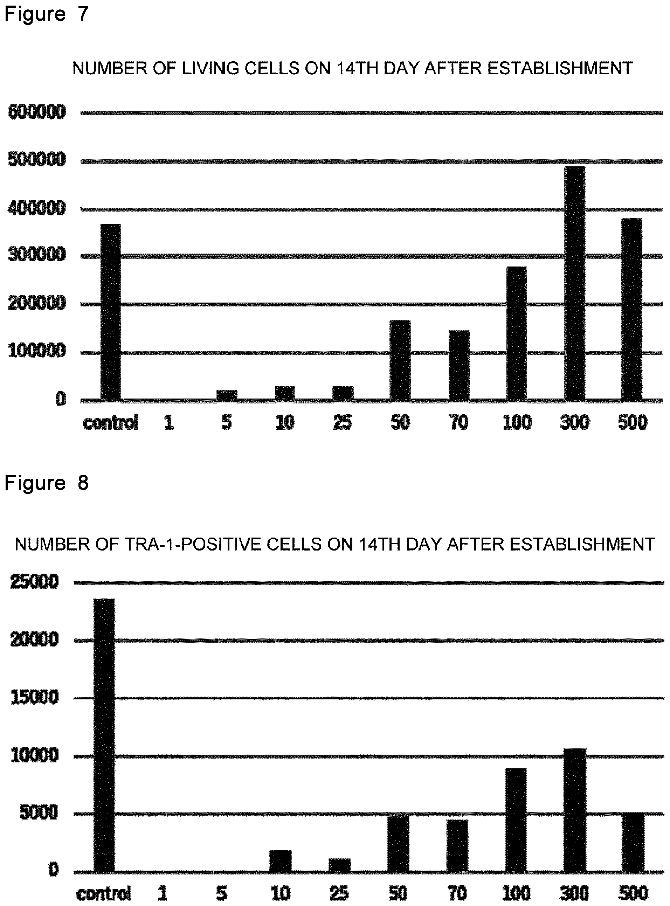

To investigate the relationship between the size of a spheroid of cells to be formed first (the number of constituent cells) and reprogramming efficiency, the number of living cells, the number of TRA-1-positive cells, and the ratio of TRA-1-positive cells on Day 14 were measured with respect to the number of PBMC cells seeded for each microwell (1 to 500 cells/microwell). Results are shown in Table 7 and Figures 7 to 9.

**[Table 7]**

| | | | | | | | | |
|---|---|---|---|---|---|---|---|---|
| Number of seeded PBMCs in each microwell | 1 | 5 | 10 | 50 | 70 | 100 | 300 | 500 |
| The obtained iPSCs in total ×10⁴cells | 0 | 1.8 | 2.9 | 16.3 | 14.4 | 27.7 | 48.9 | 38.0 |
| Number of iPSCs obtained per one seeded cell | 0 | 3.1 | 2.4 | 2.7 | 1.7 | 2.3 | 1.4 | 0.6 |

The number of iPS cells in total was high when PBMCs were gathered at 300 cells/microwell. Efficiency was high when PBMCs were gathered at 5 to 100 cells/microwell.

### [Example 3]

### Flow cytometry analysis (undifferentiation marker)

According to the method described in example 1, iPS cell lines were established using a conventional method (scaffold dependent culture) and the method of the present application (suspension conditions). The number of PBMCs seeded for each microwell was 200. iPS cells were established using blood collected from three donors (n=3). The established iPS cells were collected and treated with fixative solution (4%PFA). The iPS cells were stained with SSEA4 antibody (Alexa Fluor (R) 647 mouse anti-SSEA-4, BD Biosciences) or Oct3/4 antibody (Oct-4A (C30A3) rabbit mAb, Cell Signaling Technology) and then were measured by flow cytometry (SA3800 Spectral Cell Analyzer, Sony Corporation). Membrane transport was performed before Oct3/4 antibody staining.

Table 8 shows the result of the mean value of iPS cells derived from three donors. From this result, it is found that the degree of undifferentiation of iPS cells established by the method of the present application was superior to that of the conventional method.

**[Table 8]**

| Establishment method | Method of present application (n=3) | Conventional method (n=3) |
|---|---|---|
| SSEA4 positive cells (%) | 94.2 | 88.4 |
| OCT3/4 positive cells (%) | 97.0 | 96.4 |

### [Example 4]

### Reprogramming efficiency when cells were gathered linearly

In examples 1 to 3, cells were seeded in the container (AggreWell(TM) plate) with a microwell shaped like an inverted pyramid. Thus, cells were gathered at the tip of one side (an inclined surface that is substantially triangular in shape) of the microwell that serves as a guide portion, that is, a point at the bottom. Therefore, the reprogramming efficiency in a state other than gathering to a point, i.e., linear gathering was examined.

The reprogramming factor was introduced into PBMCs by the same method as in example 1, and the cell suspension obtained in step B10 was seeded on a low-cell-adhesion culture plate having four microwells (compartments), each with a rectangular bottom surface measuring 80x27.9 mm. The number of cells seeded in four compartments were from 2.79×10³ cells to 2.79×10⁶ cells.

The culture plate was tilted approximately 45° after seeding, and cells were gathered linearly on one side of the bottom surface (short side) in each compartment and were cultured in this state. Table 9 shows the measurement result of the number of iPS cell colonies generated in each compartment on Day 14. "Accumulated cell number" in Table 9 is a theoretical value obtained by calculating the number of cells accumulated in each compartment in the vertical direction at the beginning of culture (Day 0) when the cells were 10µm in diameter (example: 2.79×10³ cells with a diameter of 10µm were arranged horizontally in a line that measures 27.9 mm, and thus the number of accumulated cells is one in the compartment where 2.79×10³ cells were seeded).

**[Table 9]**

| PBMCs seeded for each microwell | 2.79E+03 | 2.79E+04 | 2.79E+05 | 2.79E+06 |
|---|---|---|---|---|
| accumulated cell number (vertical accumulation on Day 0) | 1 | 10 | 100 | 1000 |
| obtained iPS cell colonies | 1 | 1 | 4 | 922 |

As shown in Figure 9, iPS cell colonies were obtained for any number of accumulated cells, from 1 to 1000 cells. The number of colonies increased fourfold and 230-fold when the number of accumulated cells increased tenfold, e.g., from 10 to 100 and from 100 to 1000. Thus, it is understood that the reprogramming efficiency increased with the number of accumulated cells until the number of accumulated cells reached at least 1000. In example 2 of gathering at a point, it is assumed that iPS cells were not obtained when culture is started on a single cell (Table 7) but in linear gathering of a single accumulated cell, cells are present on both sides of the cell and thus iPS cells were obtained.

### [Example 5]

### Induction of differentiation to cardiomyocyte

The iPS cells (four lines derived from different colonies) produced by the method of example 1 were made into small pieces by using a 100-µM mesh and then were differentiation-induced to cardiomyocyte according to an established method (e.g., a method described in WO2021/172542). Specifically, after an embryoid was formed over three days, on the third day from the start of induction of differentiation, the culture medium was replaced with a StemPro-34 medium to which 1% of GlutaMAX (Invitrogen), 4×10⁻⁴ M monothioglycerol (MTG), 50 µg/ml of ascorbic acid (AA), 150 µg/ml of transferrin, 10 ng/ml of an vascular endothelial growth factor (VEGF; R&D Systems), 1 µM of IWP3 (Stemgent), 0.6 µm of dorsomorphin, and 5.4 µm of SB431542 were added, and the culture medium on the seventh day was replaced with 2 ml of a StemPro-34 medium to which 1% of GlutaMAX, 4×10⁻⁴ M MTG, 50 µg/ml of AA, 150 µg/ml of transferrin, and 5 ng/ml of VEGF were added. Thereafter, the culture medium was replaced with a culture medium having the same composition, every three to four ^{da}ys. From the start of induction of differentiation to the 12th day, cells were set in a hypoxic environment (5%O₂), and then the cells were cultured in a normal oxygen ^{en}vironment (20%O₂).

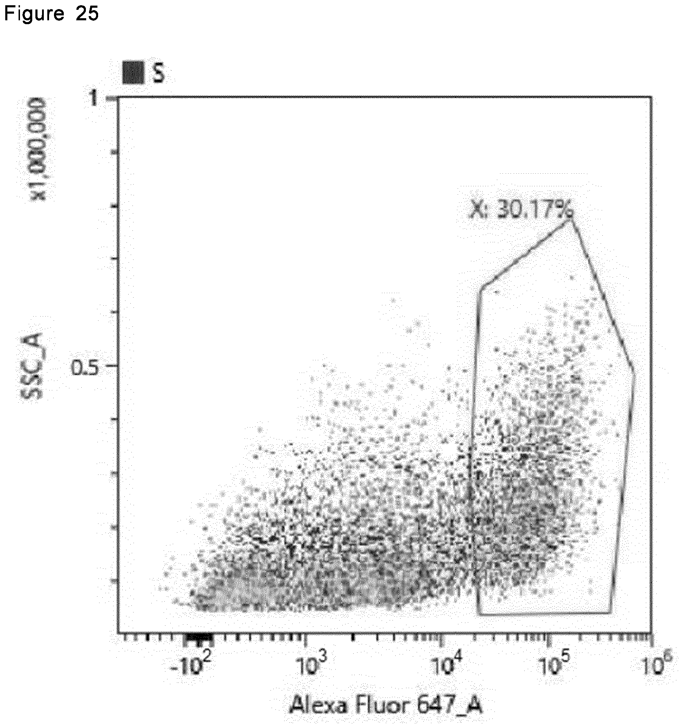

The cells were collected on the 15th day from the start of induction of differentiation, and the ratio of Troponin T-positive cells serving as a marker of cardiomyocyte was analyzed using a flow cytometer (using an anti-Troponin T antibody: Troponin T Ab-1(13-11) (Thermo Fisher Scientific, MS-295-P)). The ratio of troponin-positive cells in four lines was 6 to 30%. Figure 25 shows the result of cell lines when the ratio of troponin-positive cells was 30%.

Thus, it is found that differentiated cells (e.g., cardiomyocytes) are obtained by directly differentiation-inducing iPS cells produced by the method according to the present application.

## Claims

1. A method for producing pluripotent stem cells from somatic cells, comprising the steps of
(1) seeding somatic cells in a container having at least one compartment configured to permit gathering two or more somatic cells;
(2) bringing a reprogramming factor into contact with the somatic cells; and
(3) culturing the somatic cells in contact with the reprogramming factor in a state wherein the two or more somatic cells are gathered in the at least one compartment.

2. The method according to claim 1, wherein the compartment has a gathering portion with low cell adhesion configured to permit gathering two or more somatic cells; and
the method further comprises the step of gathering two or more somatic cells in the gathering portion.

3. The method according to claim 2, wherein the gathering step comprises at least one action selected from rotation, rocking and shaking of the container.

4. The method according to claim 3, wherein the container has a plurality of surfaces, and
the container is rotated about a rotation axis that is parallel to or intersecting a first surface having the largest area of the plurality of surfaces.

5. The method according to claim 4, wherein an angle of the rotation axis with respect to the first surface is a right angle.

6. The method according to claim 5, wherein the two or more somatic cells are gathered in a region closer to the rotation axis than the center of the container in a radial direction with respect to the rotation axis.

7. The method according to claim 5, wherein the two or more somatic cells are gathered in a region more distant from the rotation axis than the center of the container in a radial direction with respect to the rotation axis.

8. The method according to any one of claims 1 to 7, wherein the at least one compartment comprises:
a recessed portion having an opening provided on a surface of the container and a bottom surface with low cell adhesion, and
wherein the recessed portion comprises
a gathering portion configured to permit gathering two or more somatic cells; and
a guide portion configured to guide the somatic cells flowing into the container to the gathering portion.

9. The method according to claim 8, wherein the guide portion is an inclined surface which is inclined with respect to a surface of the container.

10. The method according to claim 9, wherein an angle formed between the inclined surface and the surface of the container is 15° to 60°.

11. The method according to claim 9 or 10, wherein the at least one inclined surface is configured with a flat surface.

12. The method according to claim 9 or 10, wherein the at least one inclined surface is configured with a curved surface.

13. The method according to any one of claims 9 to 12, wherein the guide portion has two or more inclined surfaces.

14. The method according to any one of claims 9 to 11, wherein the inclined surface is formed with a polygonal surface.

15. The method according to claim 14, wherein the recessed portion is shaped like a polygonal cone that is configured with three or more inclined surfaces arranged adjacent to each other and tapering from the opening to the bottom, and
the bottom surface constitutes the gathering portion, and the side surface of the recessed portion constitutes the guide portion.

16. The method according to claim 14, wherein the inclined surface is substantially triangular.

17. The method according to claim 12, wherein the recessed portion is substantially shaped like a cone tapering from the opening to the bottom, and
the bottom surface constitutes the gathering portion, and the side surface of the recessed portion constitutes the guide portion.

18. The method according to any one of claims 1 to claim 17, wherein the container comprises the two or more compartments.

19. The method according to any one of claims 1 to 18, wherein the somatic cells are floating somatic cells.

20. The method according to claim 19, wherein the floating somatic cells are blood cells.

21. The method according to claim 20, wherein the blood cells are peripheral blood mononuclear cells.

22. The method according to claim 20, wherein the blood cells comprise hematopoietic progenitor cells.

23. The method according to claim 20, wherein the blood cells are CD34-positive cells.

24. The method according to any one of claims 20 to 23, wherein the blood cells are derived from peripheral blood or cord blood.

25. The method according to any one of claims 1 to 24, wherein the somatic cells are gathered at one point in the compartment in step (3).

26. The method according to claim 25, wherein the somatic cells are seeded at 50 to 400 cells/compartment.

27. The method according to claim 26, wherein the somatic cells are seeded at 100 to 300 cells/compartment.

28. The method according to any one of claims 1 to 13 and claim 18 to 24, wherein the somatic cells are gathered linearly in step (3).

29. The method according to claim 28, wherein the somatic cells are seeded so that a theoretical value obtained by calculating the number of somatic cells accumulated on a cross section perpendicular to the linear portion is about 100 to 1,000.

30. The method according to any one of claims 1 to 29, further comprising the step of expanding somatic cells prior to bringing the somatic cells into contact with the reprogramming factor.

31. The method according to any one of claims 1 to 30, wherein in step (2) the reprogramming factor is brought into contact with the somatic cells by using a Sendai virus vector.

32. The method according to any one of claims 1 to 31, wherein the reprogramming factor is an RNA.

33. The method according to any one of claims 1 to 32, wherein the somatic cells are human somatic cells.

34. The method according to any one of claims 1 to 33, wherein step (2) is carried out after step (1).

35. The method according to any one of claims 1 to 33, wherein step (1) is carried out after step (2).

36. A container used for the method according to any one of claims 1 to 35, wherein the container has the compartment capable of gathering two or more somatic cells.

37. A container comprising
a container body capable of containing somatic cells; and
at least one compartment disposed within the container body,
wherein the compartment includes
a gathering portion with low cell adhesion capable of gathering two or more somatic cells; and
a guide portion that guides somatic cells having flown into the container to the gathering portion.

38. The container according to claim 37, further comprises a passage portion which allows the inside of the container body and the outside of the container body to communicate with each other and allows somatic cells to pass therethrough.

39. The container according to claim 37 or 38, wherein
the compartment comprises an opening provided on the inner surface of the container body and a recessed portion having a bottom surface with low cell adhesion, and
wherein the guide portion is an inclined surface constituting a part of the recessed portion and inclined with respect to the inner surface of the container body.

40. The container according to claim 38, wherein the compartment comprises an opening provided on the inner surface of the container body and a recessed portion having a bottom surface with low cell adhesion, and
the guide portion is a protruding portion which is adjacent to the opening outside the recessed portion, is located more distant from the passage portion than the opening, and protrudes from the inner surface.

41. The container according to any one of claims 36 to 40, wherein the reprogramming factor is contained within the compartment.

42. A system comprising
the container according to any one of claims 36 to 40;
a seeding device that supplies somatic cells to the container and seeds the somatic cells;
a collecting device that discharges somatic cells in contact with a reprogramming factor from the inside of the container to the outside of the container and collects the somatic cells; and
a controller that controls the seeding device and the collecting device.

43. The system according to claim 42, further comprising a tilting device that tilts a portion of the container or the entire container with respect to a reference surface to cause the two or more somatic cells in the container to gather in the compartment.

44. The system according to claim 43,
wherein the container has a flexible bottom surface, and
wherein the tilting device deforms the bottom surface to tilt the bottom surface with respect to the reference surface.

45. The system according to claim 44, wherein the tilting device tilts the container by rotating the container about a rotation axis extending in a direction orthogonal to the reference surface, or tilts a part of the container or the entire container with respect to the reference surface by pressing a part of the bottom of the container.

46. The system according to any one of claims 42 to 45, further comprising a shaking device that rocks or shakes the container enabling to cause the two or more somatic cells in the container to gather in the compartment.

47. The system according to any one of claims 42 to 46, comprising a rotating device that rotates the container about the rotation axis enabling to cause the two or more somatic cells in the container to gather in the compartment by a centrifugal force.

48. A kit for producing pluripotent stem cells from somatic cells, comprising
a container with low cell adhesion, having one or more compartments each capable of gathering two or more somatic cells; and
a reprogramming factor.

49. A program causing a computer to perform the method according to any one of claims 1 to 35.

50. A method for producing differentiated cells, comprising the steps of
providing pluripotent stem cells produced by the method according to any one of claims 1 to 35; and
culturing the provided cells in a culture medium for inducing cell differentiation.

51. The method according to claim 50, wherein the differentiated cells are cardiomyocytes.
